(19) Europäisches Patentamt
European Patent Office
Office européen des brevets

(11) EP 1 026 171 A1

(12) EUROPEAN PATENT APPLICATION

(43) Date of publication:
**09.08.2000 Bulletin 2000/32**

(21) Application number: **00105847.8**

(22) Date of filing: **23.08.1994**

(51) Int. Cl.[7]: **C07K 1/10**, C07K 1/13, A61K 49/00, A61K 47/48, A61K 9/127

(84) Designated Contracting States:
**AT BE CH DE DK ES FR GB GR IE IT LI LU MC NL PT SE**

(30) Priority: **24.08.1993 GB 9317618**

(62) Document number(s) of the earlier application(s) in accordance with Art. 76 EPC:
**94924920.5 / 0 714 402**

(71) Applicant:
**PolyMASC Pharmaceuticals plc**
**London NW 3 2EZ. (GB)**

(72) Inventors:
- **Francis, Gillian Elizabeth**
  **Berkshire, RG4 9GH (GB)**
- **Fisher, Derek**
  **Hertfordshire, AL3 4HY (GB)**
- **Delgado, Christina**
  **London, NW6 3DY (GB)**
- **Malik, Farooq**
  **London, SW 16 2TS (GB)**

(74) Representative:
**Nachshen, Neil Jacob et al**
**D Young & Co**
**21 New Fetter Lane**
**London EC4A 1DA (GB)**

Remarks:
This application was filed on 20 - 03 - 2000 as a divisional application to the application mentioned under INID code 62.

# (54) Polymer modification

(57) A process for producing an adduct of a polymer and a target material which process comprises the steps of

(a) reacting tresyl chloride with a polymer of formula (II)

$$(C)_c \text{ POL - } G_g \qquad (II)$$

wherein

POL is a polymer moiety of valency c+g,
C is a capping group and c is zero or a positive number and
G is a terminal hydroxyl group reactive with the compound of formula (I) and g is a positive number

so as to form

a sulphonate ester-activated polymer of formula (III)

$$(C)_c \text{ POL - (tresyl)}_g \qquad (III)$$

wherein
C, POL, -AM, c and g are as defined above

(b) reacting the activated polymer of formula (III) with the target material and

(c) recovering the adduct of the polymer and the target material,
in which process:

(i) the polymer of formula (II) is dry as determined by benzene distillation, and
(ii) the reaction of the tresyl chloride with the polymer of formula (II) is conducted in an organic solvent which is inert to the reagents and to the product of formula (III) and is anhydrous as obtainable using molecular sieves of 0.3nm;
(iii) the reaction of the tresyl chloride with the polymer of formula (II) is conducted in a reaction vessel from which water is excluded;
(iv) the activated polymer of formula (III) so produced is recovered and either used directly in step (b) or stored, prior to use in step (b), in the presence of a desiccating agent more hygroscopic than the product of formula (III); and
v) the reaction of the activated polymer with the target material is conducted in a non-denaturing medium and non-denaturing temperature with respect to the target material.

EP 1 026 171 A1

## Description

The present invention relates to a process for direct covalent bonding of polymer moieties to target molecules in order to improve or modify the biological properties of the target molecules. The invention also relates to certain products which may be produced by this process and to intermediates useful in the process as well as to methods of use of the products of the process.

Covalent attachment of polyethylene glycol(PEG) and other polymers to proteins is well known to convey many benefits which improve the pharmacological and some physiological properties of proteins (reviewed in Nucci at al (1991) Advanced Drug Delivery Reviews, **6**, 133-151; Zalipsky & Lee (1992) Biomedical Applications of Polyethylene Glycol Chemistry (ad. by J.M. Harris), Plenum, New York; Fuertges & Abuchowski, Journal of Controlled Release, **11**, 139-149 1990).

There are many methods for achieving covalent coupling of polymers like PEG to proteins. All consist of a method of activating the polymer by attachment of a group or groups herein called an "activating moiety" or by coverting a terminal moiety of the polymer into an "activating moiety" and a second step where the polymer couples to the target molecule, usually via a residual portion of the "activating moiety" herein called the "coupling moiety".

Typical targets are proteins which contain one or more reactive groups on which the attachment of the coupling moiety or polymer itself can take place, such as primary and secondary amino groups, thiol groups and aromatic hydroxy groups. Alternatively, the protein molecule has been modified so that it contains suitable groups. Similar techniques are applied in coupling PEG and other polymers to other types of target molecule.

Examples of known techniques are:

Cyanuric Chloride Methods: USP 4 179 337 Davis et al; USP 4 301 144 Iwashita & Ajisaka; USP 4 261 973 Lee & Sehon; EP-A-0 098 110 Hiratani; USP 4 495 285 Shimitzu; USP 4 609 546 Hiratani; WO 86/04145 Tomasi; EP-A-0 210 761 Miyata et al; WO 90/06952 Ishikawa et al; GB 2 238 959A Sehon et al; Abuchowski et al (1977a), Journal of Biological Chemistry, **252**, 3578-3581, Abuchoweki et al (1977b), Journal of Biological Chemistry, **252**, 3582-3586.

Succinimidyl Active Ester Methods: Boccu et al (1983) Z. Naturforsch, **38c** 94-99; Abuchowski et al (1984) Cancer Biochem. Biophys., **7**, 175-186; Leonard et al (1984) Tetrahedron, **40**, 1585-1590; USP 4 412 989 Iwashita et al; WO 86/04145 Tomasi; WO 87/00056 Katre & Knauf; EP-A-0 247 860 Katre et al; EP-A-0-260 098 Inada et al; WO 89/06546 Shadle et al; Katre et al (1987) Proc. Natl. Acad. Sci. USA, **84**, 1487-1491.

Carbonyldiimidazole Method: Beauchamp et al (1983) Analytical Biochemistry, **131**, 25-33; EP-A-0 154 432 Sawai et al.

Phenylchloroformate Methods: Veronese et al (1985) Appl. Biochem. Biotechnol., **11**, 141-152; WO 89/06546 Shadle et al; WO 90/15628 Groves et al.

PEG-Succinate Mixed Anhydride Methods: Ahlstedt et al (1983) Int. Arch. Allergy Appl. Immunol., **71**,228-232; Richter and Akerblom (1983) Int. Arch. Allergy Appl. Immunol, **70**, 124-131; Richter & Akerblom (1984) Int. Arch. Allergy Appl. Immunol, **74**, 36-39; Lee and Sehon (1978) Immunol. Rev, **41**, 200-247; USP 4 261 973 Lee and Sehon.

Organic Sulphonyl Halide Methods: USP 4 415 665 Mosbach & Nilsson; Delgado et al (1990) Biotechnology and Applied Biochemistry, **12**, 119-128; WO 90/04650 Francis at al; WO 90/04606 Delgado at al; WO 90/04384 Fisher.

PEG-Aldehyde Methods: Harris at al (1989) Separations Using Aqeous Phase Systems. Applications in Cell Biology and Biotechnology (ad. by D. Fisher and I.A. Sutherland), p.203. Plenum Press, London; Harris at al (1991) Water-Soluble Polymers (ad. by S.W. Shalaby, C.L. McCormick and G.B. Butler), American Chemical Society, Washington, D.C. USP 4 002 531 foyer; EP-A-0-251 304 Minami at al; WO 90/05534 Capon at al.

PEG-Maleimide and Related Methods: Goodson & Katre (1990) Biotechnology, **8**, 343-346.

Phenylglyoxal Method: EP-A-0 340 741 Meeda at al.

Succinimide Carbonate Method: WO 90/13540 Zalipsky; WO 91/07190 Nho at al;

Cyanogen Bromide Method: USP 4 301 144 Iwashita & Ajisaka.

Poly-PEG Maleic Acid Anhydride Method: Yoshimoto et al (1987) Biochemical and Biophysical Research Communications, **148**, 876-882.

The key features of the methods are summarised in Table 1.

Table 1

| Method | Coupling Moiety (Boxed) and general structure of adduct | Bond | Co-Product | Recommended Coupling Reaction |
|---|---|---|---|---|
| Cyanuric Chloride | 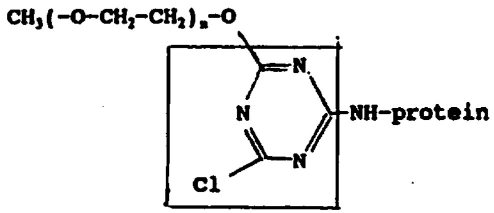 | | HCl | 1h, RT[2], pH 9.2(3)<br>1h, RT, pH 9.8 (4)<br>1h, 37°C, pH10(5) |
| Succinimidyl active ester (succinimidyl succinate) | 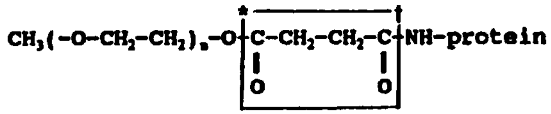 | †amide bond<br>*ester bond | N-hydroxy-succinimide | 30 min, pH 7(6)<br>1h, RT, pH8.25(7)<br>30min, RT, pH9(8) |
| PEG-succinate mixed anhydride | 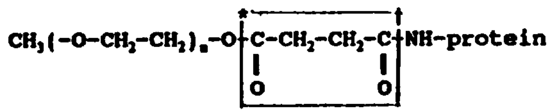 | † amide bond<br>* ester bond | ester plus ester-modified protein | 30 min, RT plus overnight 0°C pH 8.7 (9) |
| Phenylchloro-formates | 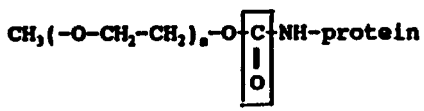 | carbamate | substituted phenol[1] | 1-5h, pH8.3-9.3(10) |
| Carbonyldiimi-dazole | 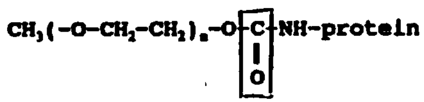 | carbamate | imidazole | 24-72h, 4°C, pH8.5 (11,12) |

| Method | Coupling Moiety (Boxed) and general structure of adduct | Bond | Co-Product | Recommended Coupling Reaction |
|---|---|---|---|---|
| Succinimide carbonate | 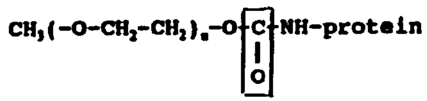 | carbamate | N-hydroxy-succinimide | 30min,pH9.3 (13) |
| Poly(PEG-MA) anhydride | 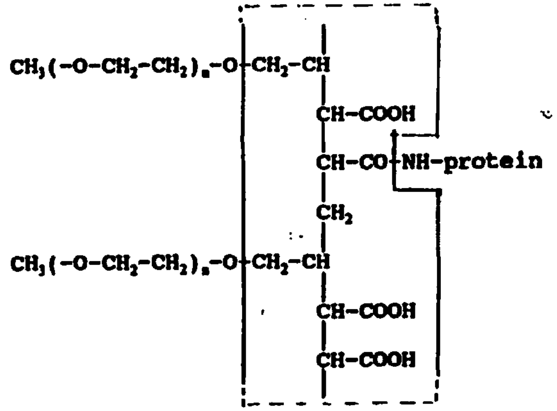 | †amide bond | | 2h,25°C,pH8.5 (14) |
| PEG-maleimide | 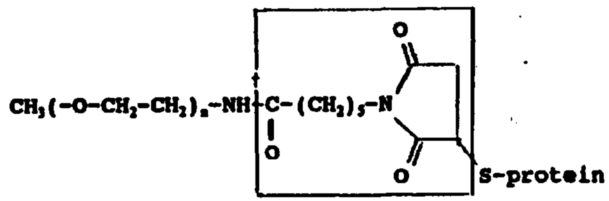 | †amide bond | | several hours,RT, pH5-7 (15)<br>2hr, RT, pH5.5 (16) |

| Method | Coupling Moiety (Boxed) and general structure of adduct | Bond | Co-Product | Recommended Coupling Reaction |
|---|---|---|---|---|
| PEG-acetaldehyde | no coupling moiety present or ethylene oxide unit<br>$CH_3(-O-CH_2-CH_2)_n\boxed{-O-CH_2-CH_2-}$‡NH-protein | ‡ secondary amine | oxidised cyano-borohydride | Not indicated (17) |
| Sulphonic halogenide | no coupling moiety present<br>‡<br>$CH_3(-O-CH_2-CH_2)_n$-NH-protein | ‡ secondary amine | sulphonic acid | 1h,RT,pH 7.5 (18) |
| Phenyl glyoxal | 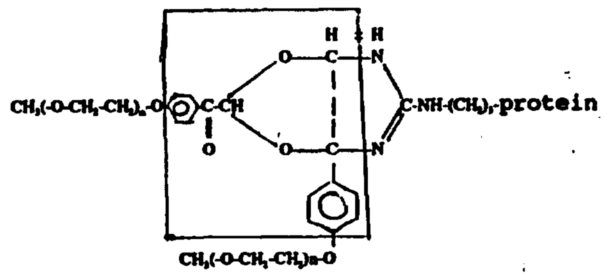 | ‡ secondary amine | | pH8.5,7hr,RT (19) |

| Method | Coupling Moiety (Boxed) and general structure of adduct | Bond | Co-Product | Recommended Coupling Reaction |
|---|---|---|---|---|
| Cyanogen bromide | This process potentially provides three products<br>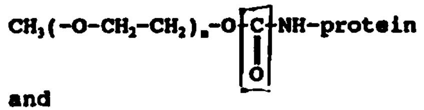<br>and<br>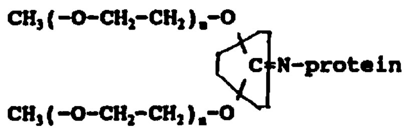<br>and<br>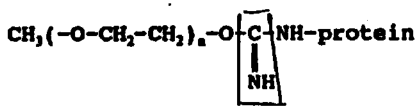 | carbamate<br><br>imido carbonate<br><br>- | $NH_3$<br><br>$NH_3$<br><br>None | 16h,0°C,pH7.5 (20) |
| Amine acylation | 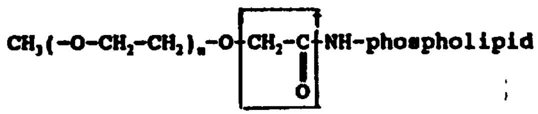 | † amide | PEG-carboxylate | 3h,65°C, benzene (21) |

| Method | Coupling Moiety (Boxed) and general structure of adduct | Bond | Co-Product | Recommended Coupling Reaction |
|---|---|---|---|---|
| PEG-propion-aldehyde | $CH_3(-O-CH_2-CH_2)_n$-[$S-(CH_2)_3-S-(CH_2)_3$]‡-NH-protein<br>or<br>$CH_3(-O-CH_2-CH_2)_n$-[$O-CH_2-CH_2-CH_2$]‡-NH-protein | ‡ secondary amine | oxidised cyano-borohydride | 1H, RT, pH9 (22) |

Notes

1. cf. 2,4,5-trichlorophenol from MPEG 2,4,5-trichlorophenylcarbonate; p-nitrophenol from MPEG-p-nitrophenylcarbonate.
2. Room temperature
3. Abuchowski (1977a)
4. Pyatak <u>et</u> <u>al</u>. (1980) Res, Commun. Chem. Pathol. Pharmacol., 29, 113-127.
5. Yoshimoto, <u>et</u> <u>al</u>. (1986) Jpn. J. Can. Res., 77, 1264-1270.
6. Abuchowski (1984).

**Notes to Table 1 cont'd**

7. Leonard (1984).
8. Katre (1987).
9. Wie et al. (1981) Int. Arch. Allergy Applied Immunol., 64,84
10. Veronese (1985).
11. Beauchamp (1983) and EP-A-0 154 432
12. Pizzo (1991) Advanced Drug Delivery Reviews, 6, 153-166.
13. Zalipsky (1992).
14. Yoshimoto (1987).
15. Aldwin and Nitecki, (1987) Analytical Biochemistry, 164, 494-501.
16. Goodson and Katre (1990).
17. Harris (1989).
18. Delgado (1990).
19. Maeda et al. (1989) EP-A-0340741
20. Iwashita et al. (1981) US-A-4301144
21. Sears (1983) EP-A-0072111
22. Harris et al. New polyethylene glycols for biomedical applications, in Shalaby SW, McCormick CL, Butler GB (eds): Water-soluble Polymers, Washington D.C., American Chemical Society, 1991.

Methods previously disclosed suffer from one or more of the following defects:

1. Substantial loss of biological activity (e.g. 20-95% loss of bio-activity) is frequently seen with the cyanuric chloride and carbonyldiimidazole methods and occasionally with phenylchloroformate and succinimidyl active ester methods.
2. The coupling of PEG (or other polymers) to proteins (or other target molecules) is, with few exceptions, in a manner which leaves part of the activating moiety, the coupling moiety, between the polymer and the target molecule. Of the above methods, only the organic sulphonyl halide methods and a PEG-acetaldehyde method (one of the PEG aldehyde methods [Harris (1989), Harris (1991)]) couple PEG directly without coupling moieties (see Table 1). With the exception of some PEG acetaldehyde methods where the coupling moiety is ethylene oxide (and thus indistinguishable from PEG itself) and the direct coupling methods above, all other coupling methods incorporate a coupling moiety distinct from the polymer and the target and are regarded herein as "indirect" coupling methods.
The incorporation of a coupling moiety generates further problems depending on the nature of the coupling moiety, thus

(i) some coupling moieties provide reactive groups capable of linking further molecules to the polymer-target construct via the coupling moiety (e.g. the triazine ring of the cyanuric chloride method, Leonard, M. et al., Tetrahedron, 40: 1585 (1984));

(ii) some coupling moieties provide an immunogenic/antigenic group (e.g. the triazine ring of the cyanuric chloride method);

(iii) some coupling moieties are potentially toxic or are themselves of unknown toxicity but derived from a compound known to be toxic (e.g. the triazine ring of the cyanuric chloride method and reagents in the phenylchloroformate method); and

(iv) some coupling moieties provide targets for enzymatic cleavage (see below).

3. Coupling in some instances is via an unstable bond liable to be cleaved by enzymes available in serum, plasma, cells or other biological materials or by procedures applied to the polymer-target product. This has two possible deleterious consequences,

(i) the polymer-target construct is degraded enzymatically or by the conditions required for subsequent reaction steps; the former occurs with methods generating amide and/or ester bonds (see Table 1); and

(ii) removal of the polymer alters the target molecule; this occurs with some succinimidyl active ester and mixed anhydride methods,

and either or both of these can occur.

4. Many methods recommend long coupling times and/or unphysiological pH, thus rendering some target molecules less active or inactive (cf. the carbonyl-diimidazole, cyanuric chloride, phenylchloroformate and some succinimidyl active ester methods (see Table 1)).

5. Many methods use activated polymer species and/or produce co-products which are toxic in a wide range of bioassays and which are potentially toxic in vivo if not separated from the product (e.g phenylchloroformate, cyanuric chloride methods).

6. Many methods produce products highly contaminated with either activated or inactivated polymer, causing problems in analytical methods like FPLC and toxicity in some bioassay systems (cf. the organic sulphonyl halide [Delgado (1990)] and phenylchloroformate [Veronese (1985)) methods).

7. Some methods are unsuitable for use in aqueous solution, thus limiting the target molecules to those which will tolerate non-aqueous conditions (cf. the organic sulphonyl halide method using trifluoromethanesulphonyl chloride).

8. Some of the activated polymer constructs are unstable, for instance being subject to hydrolysis during either the activation or coupling reactions (cf. the phenylchloroformate method [Veronese (1985)]). The PEG-acetaldehyde is sensitive to decomposition under basic conditions and can give irreproducible results [Harris et al. (1991)].

The present inventors have previously described PEGylation by an organic sulphonyl halide method using 2,2,2-trifluoroethanesulphonylmonomethoxy PEG (TMPEG) as follows:

1. WO 90/04606 discloses a method for fractionation of PEG-proteins formed by the TMPEG method (discussed below) and an individual adduct, PEG-gm-CSF. The fractionation method is unrelated to the present invention. The PEG-protein products of the processes disclosed in this application, and specifically disclosed materials such as the PEG-gm-CSF adduct, are excluded from the present invention.

2. PCT WO 90/04650 discloses a method to separate DNA/protein complexes using PEG adducts formed by the TMPEG in which PEG acts as an affinity ligand. These complexes are also excluded from the present invention.

3. PCT WO 90/04384 discloses the TMPEG method for attaching PEG to liposomes. Such PEGylated liposomes are excluded from the present invention. It should be noted that the objective of coupling PEG to lipsomes in this patent was to improve half life and liposome stability.

The TMPEG method mentioned above, for instance in WO-A-90/04606, comprises activation of monomethoxy PEG (MPEG) with 2,2,2-trifluoroethanesulphonyl chloride (tresyl chloride) to produce tresyl MPEG (TMPEG) which is subsequently reacted with the target protein molecule to produce monomethoxy products. The same technique is described in WO 90/04650 for coupling monomethoxy PEG moieties to DNA/protein complexes and in WO/04384 for coupling monomethoxy PEG moieties to liposomes. Further investigation of the TMPEG method has revealed that the reactivity of the activated polymer, TMPEG, is severely impaired necessitating the use of uneconomically large ratios of TMPEG to the target molecules and has exposed the rather poor yield of the final products and the low purity thereof, necessitating extensive purification and thereby further reducing the overall yield.

The present inventors have addressed these difficulties in further research and have identified a number of factors which, in combination, contribute to the poor performance of the TMPEG process. This has allowed development of an improved technique for producing PEG adducts. The technique has been extended to include additional

technical developments permitting more facile formation of complex adducts of PEG and other polymers and the formulation of adducts of a wider range of target molecules.

The present invention relates to an improved process for producing polymer:target molecule adducts which are directly covalently linked without any intervening coupling moiety residue from the activating group on the polymer and in which the link between polymer and target moieties is non-immunogenic, non-antigenic, non-toxic and non-biodegradable, this objective being achieved by careful control of the properties of certain of the reagents and the reaction conditions. The coupling step may be performed in aqueous media and occurs rapidly thus minimising damage to fragile target species.

Accordingly the invention provides a process for producing adducts of a polymer and a target material which process comprises the steps of

(a) reacting either

(i) an activating compound of formula (I)

$$X\text{-}AM \qquad (I)$$

wherein

-AM is an activating sulphonyl ester moiety optionally bearing a group for covalent bonding to a solid support, the solvolysis substituent constant of the group -AM being less than that of the trifluoromethane sulphonate group, and
X is a leaving group

or
(ii) reacting a solid support bearing moieties of formula (I')

$$X\text{-}AM'\text{-} \qquad (I')$$

wherein

-AM'- is activating sulphonyl ester moiety covalently bound to the solid support, the group -AM'- being such that the solvolysis substituent constant of the group -AM'- is less than that of the trifluoromethane sulphonate group, and
X is as defined above with a polymer of formula (II)

$$(C)_c\ POL - G_g \qquad (II)$$

wherein
POL is a polymer moiety of valency c+g,
C is a capping group and c is zero or a positive number and
G is a terminal hydroxyl group reactive with the compound of formula (I) and g is a positive number

so as to form

(i) a sulphonate ester-activated polymer of formula (III)

$$(C)_c\ POL - (AM)_g \qquad (III)$$

wherein

C, POL, -AM, c and g are as defined above and the group(s) -AM are linked to termini of the polymer as sulphonate esters of terminal hydroxyl groups

or
(ii) a solid support-bound, sulphonate ester-activated polymer of formula (III')

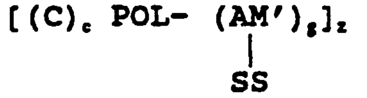

(III')

wherein
C, POL, -AM'-, c and g are as defined above,
SS is a solid support and z is the number of sulphonate ester-activated polymer moieties on the solid support and the groups -AM'- are linked to the termini of the polymer as sulphonate esters of terminal hydroxyl groups,

and, when -AM bears a group for covalent bonding to a solid support, reacting the sulphonate ester-activated polymer of formula (III) with a solid support to form a solid support-bound, sulphonate ester-activated polymer of formula (III') as defined above
(b) reacting the sulphonate ester-activated polymer of formula (III) or (III') with the target material and
(c) recovering the adduct of the polymer and the target material,
in which process:

(i) the polymer of formula (II) is dry as determined by benzene distillation,
(ii) the reaction of the compound of formula (I) or (I') with the polymer of formula (II) is conducted in an organic solvent which is inert to the reagents and to the product of formula (III) or (III') and is anhydrous as obtainable using molecular sieves of 0.3nm;
(iii) the reaction of the compound of formula (I) or (I') with the polymer of formula (II) is conducted in a reaction vessel from which water is excluded;
(iv) the sulphonate ester activated polymer of formula (III) or (III') so produced is recovered and either used directly in step (b) or stored, prior to use in step (b), in the presence of a desiccating agent more hygroscopic than the product of formula (III) or (III'); and
(v) the reaction of the sulphonate ester-activated polymer with the target material is conducted in a non-denaturing medium and non-denaturing temperature with respect to the target material.

The compounds of formula (I) and solid supports bearing moieties of formula (I'), the polymer moiety POL in the polymers of formulae (II), (III) and (III'), the sulphonate ester-activating moieties -AM and -AM'-, the reactive groups X and G and the capping groups C will be described in more detail below. First, some variants of the overall process will be described:

In general the process of the invention is conducted in the liquid phase, using a compound of formula (I) in step (a), in solvents and reaction media as defined above. However it is also possible to conduct the reaction at a solid:liquid interface by using a solid support-bound, sulphonate ester-activated polymer of formula (III'). In this latter case the reaction can be conducted on, for instance, a column of solid support-bound sulphonate ester-activated polymer with other reagents and reaction media being passed through the column.

The objective of performing the reaction on a solid support is that although the target is exposed to a molar excess of sulphonate ester-activated polymer, only those polymer moieties which become attached to the target are eluted from the solid support and the potentially toxic coproduct remains attached to the column. The flow rate and column length provide a means of controlling the degree of modification. Column media can also be selected to achieve differential elution of targets modified to different extents.

Binding of the sulphonate ester-activated polymer to the solid support is achieved <u>via</u> the activating ester-sulphonyl moiety -AM in one of two ways:

1. The process is conducted using a compound of formula (I) which is an activating sulphonyl compound having a binding substituent for later linking to the solid support. In this variant, the reaction between the compound of formula (I) and the polymer of formula (II) is conducted in the solution phase and is followed by the step of binding the product of formula (III) to the solid support. The reaction in step (b) between the solid support-bound, sulphonate ester-activated polymer of formula (III') and the target material is then performed at the solid:liquid interface.
2. The process is conducted using a solid support bearing activating sulphonyl moieties of formula (I') in step (a). In this case the reaction of the compound of formula (I') with the polymer material of formula (II) is conducted at the solid:liquid interface and directly forms the solid support-bound, sulphonate ester-activated polymer of formula (III'). The solid support bearing moieties of formula (I') is produced by reacting a compound of formula (I) or a precursor therefor which bears a group for covalent bonding to a solid support with the solid support as a preliminary to step (a) of the present process. Step (b) is again conducted at the solid:liquid interface as in (1) above.

The benefit of conducting the reaction(s) at a solid:liquid interface is primarily seen in the greater purity of the products since unreacted sulphonate ester-activated polymer is left on the solid support as are the reaction products other than the polymer:target adduct.

The present invention therefore provides the following particular embodiments of the process:

1. A process as described above comprising the steps of reacting a compound of formula (I) with a polymer of formula (II) in the liquid phase to produce a sulphonate ester-activated polymer of formula (III) and reacting the sulphonate ester-activated polymer with the target material in the liquid phase.
2. A process as described above comprising the steps of reacting a compound of formula (I) wherein -AM is an activating sulphonyl moiety having one or more groups for covalent bonding to a solid support with a polymer of formula (II) in the liquid phase, coupling the thus-produced sulphonate ester-activated polymer to a solid support material via the binding groups of the moieties -AM and reacting the target material with the thus-produced solid support-bound, sulphonate ester-activated polymer. In this embodiment step (c) is achieved when the adduct is released from the solid support, eluted and thus recovered.
3. A process as described above comprising the steps of reacting a solid support bearing moiety of formula (I') with a polymer of formula (II) so as to form a solid support-bound, sulphonate ester-activated polymer of formula (III') and reacting the solid support-bound, sulphonate ester-activated polymer with the target material.

In further variants of the process of the invention, which can be conducted in conjunction with any of the foregoing variants, the proportion of the groups -AM on the sulphonate ester-activated polymer which react with the (initial) target material is controlled such that in at least a proportion of the molecules of polymer:target adduct produced there remain unreacted activating sulphonyl moieties -AM. Such unreacted reactive groups -AM may then be reacted with one or more different further target materials so as to form materials polymer:$[target]_n$ wherein n is 2 or more and the initial and further target entities are different; or they are reacted with more of the initial target material so as to form materials polymer:$[target]_n$ wherein the target entities are all the same; or they are reacted with further unreacted groups in the initial target entity of the polymer:target adduct (for instance under conditions which hinder intermolecular reactions, such as high dilution) such that the polymer moieties are bonded at two or more positions to the target entity.

It will be appreciated that, since the polymer and/or the target materials may be multivalent, it is possible by the process of the invention to produce a variety of polymer:target structures. By way of example, a univalent polymer and univalent target produce 1:1 adducts; a bivalent target and a univalent polymer may form adducts wherein the target entities bear two polymer moieties whereas a bivalent polymer and a univalent target may produce species where two target entities are linked to a single polymer moiety; use of higher-valent polymers can lead to the formation of clusters of target entities bound to a single polymer moiety whereas higher-valent targets may become encrusted with a plurality of polymer moieties. In general the target moieties are likely to have more than one reactive group which will react with the activated polymer and the possibility of forming complex structures must always be considered; when it is desired to form simple structures such as 1:1 adducts of polymer and target, or to use bivalent polymers to form target:polymer:target adducts, it will be necessary to use predetermined ratios of activated polymer and target material, predetermined concentrations thereof and to conduct the reaction under predetermined conditions (such as duration, temperature, pH etc.) so as to form a proportion of the described product and then to separate the described product from the other reaction products.

The necessary reaction conditions, proportions and concentrations of the reagents can be obtained by relatively simple trial-and-error experiments with appropriate scaling-up as necessary. It will be appreciated that molar proportions and the concentrations of individual reagents may need to be adjusted on scaling up compared with the optimal molar proportions and concentrations identified at the laboratory bench scale for production of a particular product; this may also require relatively simple trial-and-error experimentation. Purification and separation of the products is similarly achieved by conventional techniques well known to those skilled in the art.

The present invention therefore provides the following particular embodiments of the process:

1. A process as described above comprising, in step (b), reacting, in predetermined molar ratio and at predetermined concentrations, sulphonate ester-activated polymer having two moieties -AM with a first target material, so as to produce a sulphonate ester-activated 1:1 adduct of polymer and target material, then reacting the 1:1 adduct with a second target material so as to produce a $target^1$:polymer:$target^2$ adduct, wherein the first and second target materials may be the same or different, and then recovering the adduct in accordance with step (c).
2. A process as described above comprising, in step (b), reacting, in predetermined molar ratio and at predetermined concentrations, sulphonate ester-activated polymer having only one moiety -AM or -AM'- with a target material having more than one reactive group so as to form an adduct of the polymer and target having a preselected number of polymer molecules per molecule of target or, especially for macromolecular targets having a large number of reactive groups, so as to form an adduct in which a preselected proportion of the reactive groups have

been reacted with and linked to polymer molecules, and then recovering the adduct in accordance with step (c). The preselected number may be, for instance from 1 to 10, preferably from 1 to 5, for example 1, 2, 3 or 4. The preselected proportion may be, for instance from 1 to 100 %, preferably from 5 to 95 %, for example, 10, 20, 30, 40, 50, 60, 70, 80, or 90%.

3. A process as described above comprising, in step (b), reacting, in predetermined molar ratio and at predetermined concentrations, sulphonate ester-activated polymer having two or more activating sulphonyl moieties -AM with a target material having two or more reactive groups so as to produce a sulphonate ester-activated 1:1 adduct of polymer and target then changing the reaction conditions to hinder intermolecular reactions whilst permitting intramolecular reactions between groups -AM on the polymer and reactive groups on the target so as to form adducts of polymer and target having two or more covalent bonds between the polymer and target moieties, and then recovering the adduct in accordance with step (c).

These process variants may each be used in conjunction with any of the process variants described earlier relating to liquid phase and solid:liquid phase interface reactions.

The reagents used in the process of the invention will now be described starting with the compounds of formula (I):

ACTIVATING COMPOUNDS OF FORMULA (I) and SOLID SUPPORTS BEARING MOIETIES OF FORMULA (I')

The leaving group X is intended to react with groups G on the polymer of formula (II) and, in the broadest sense, may be selected from any of the known leaving groups which will undergo such a reaction. However it is generally preferred that X is a halogen and most preferably X is chlorine.

Activating moieties, -AM and -AM'- are sulphonyl-type activating groups, and optionally have a binding substituent for subsequent linking to a solid support or are attached to a solid support. The groups -AM will be described below in general, followed by further information on binding substituents and linkage to solid supports.

The activating moieties -AM are moieties having the following properties:

1. They are sulphonyl groups; the derived sulphonate esters are a large class of compounds with a broad range of solvolytic activities.

2. They have appropriate electron withdrawing groups on the sulphur. The suitability of such groups can be estimated by linear free energy relationship plots. It has been observed [Crossland et al (1971) J.Amer.Chem.Soc., **93**, 4217-4219) that substituent constants may be applied to the reactivity of groups such as 2,2,2-trifluoroethanesulphonate (tresylate), toluenesulphonate (tosylate), and methanesulphonate (mesylate) groups; there is a correlation observed in a Hammett plot between the rates of solvolysis of sulphonate ester and the nature of the substituent on the sulphur and although this correlation is only approximate, it allows the predication of gross effects of various sulphonyl substituents on solvolytic reaction rates. This technique provides a distinction between those sulphonate ester groups which are too highly reactive to be of use in the process of the present invention (i.e. those having a substituent constant or solvolysis rate equal to or greater than that of the trifluoromethane sulphonate (triflate) group) and those which are of use and provides a useful means of preliminary selection within the latter. Substituent constants must be less than the values for the trifluoromethyl group in trifluoromethanesulphonates, preferably they are equal to or greater than the values of the tosyl group in tosylates [Crossland et al (1971)]. This can for example be achieved by the inclusion of fluorine atoms in alkyl substituents as in 2,2,2-trifluoroethanesulphonate (tresyl) esters. By selecting -AM from compounds having this range of substituent constants, one is selecting reagents with a predetermined range of reactivities. This gives a means of predicting: a) the rate of the coupling reaction and b) the tendency of -AM to undergo hydrolysis in aqueous solution. It is important that the two rates are below that of triflate and preferably equal to or above that of tosylate since, if the reactivity is too low, the coupling time will be long with potential damage to the target and if it is too high, hydrolysis will compete significantly with the coupling reaction and may preclude its occurring adequately in aqueous solution.

If the target molecule is one that can withstand organic solvents these may be used for the coupling reaction. However, where the target molecule can withstand a slow coupling reaction then compounds with lower Hammett substituent constants than the range given above can be used.

3. They contain no groupings more susceptible to nucleophilic attack than the terminal carbon atom of the polymer moiety for instance PEG, or equivalent atom of any other polymer moiety (i.e. the carbon or equivalent atom bonded to the oxygen of the sulphonate group of the group -AM). The consequence of this is that no "coupling moiety" (i.e. residual part of the activating moiety) lies between the polymer and the target molecules.

4. The compounds from which the groups -AM are derived should preferably be stable.

5. To minimise toxic effects, the groups -AM and the compounds from which they are derived should preferably be of low toxicity in vitro and in vivo.

6. The compounds from which the groups -AM are derived should be readily separated from the product of formula (III).

Preferred groups -AM are:

2,2,2-trifluoroethanesulphonyl,
pentafluorobenzenesulphonyl,
fluorosulphonyl,
2,4,5-trifluorobenzenesulphonyl,
2,4-difluorobenzenesulphonyl,
2-chloro-4-fluorobenzenesulphonyl,
3-chloro-4-fluorobenzenesulphonyl,
4-amino-3-chlorobenzenesulphonyl,
4-amino-3-fluorobenzenesulphonyl,
o-trifluoromethylbenzenesulphonyl,
m-trifluoromethylbenzenesulphonyl,
p-trifluoromethylbenzenesulphonyl,
2-trifluoromethoxybenzenesulphonyl,
4-trifluoromethoxybenzenesulphonyl, and
5-fluoro-2-methylbenzenesulphonyl groups.

The most preferred group -AM is 2,2,2 trifluoroethane-sulphonyl.

It should be appreciated that the definition of the activated polymers used in the present invention excludes certain sulphonic acid esters of polymers which have previously been used in "active ester" methods of PEGylation such as the ester between the carboxylic acid of the polymer and the hydroxyl of 4-hydroxy-3-nitrobenzene sulphonic acid described in Katre et al.(1987).

The binding substituents for linking the group -AM to a solid support are suitably groups which can be coupled to conventional solid support materials, optionally via a conventional spacer moiety, by well known techniques. A preferred binding substituent is an aminophenyl group which can be coupled to a solid support via the amino substituent. Preferred compounds of formula (I) for binding to solid supports are therefore sulphonyl halides having an aminophenyl group bound to the sulphur atom, possibly via other moieties, such as 2',3',5',6'-tetra-fluoro-4-amino-phenyl-azobenzene-4'-sulphonic halides, especially the chloride.

Solid support materials which may be used in the process of the invention are entirely conventional and well known to those skilled in the art. It is preferred that the solid support is selected from silica, silconised glass, agarose, Sepharose, polystyrene divinyl benzene copolymer and polyacrylamide.

Compounds of formula (I) and solid supports bearing groups of formula (I') may be produced by conventional techniques well known to those skilled in the art. Many of these compounds are commercially available.

Compounds of formula (I) which are intended for linking to solid supports are also readily available or are produced by conventional techniques; they may be coupled to solid supports by conventional techniques optionally with introduction of spacer groups. Alternatively, in view of the reactivity of sulphonyl halides, it may be preferred to couple a precursor, such as a sulphonic acid, of the compound of formula (I) with the solid support or reagents for introducing a spacer group then to convert the bound precursor to the sulphonyl halide group.

An example of a compound of formula (I) bearing a group for covalent bonding to a solid support is tetrafluoro-4-amino-azobenzene-4'-sulphonyl chloride which can be generated in a three step procedure as described by Scouten et al (1991) Journal of Chromatography, **376**, 289-298 as follows:-

1. Tetrafluorosulphanilic acid can be prepared from tetrafluoroaniline.
2. Tetrafluoro-4-amino-azobenzene-4'-sulphonic acid is prepared from tetrafluorosulfanilic acid by diazotisation and reaction with aniline.
3. The sulphonic acid is converted to the sulphonyl chloride by drying with thionyl chloride and then grinding with phosphorus pentachloride and phosphorus oxychloride.

Coupling of compounds of formula (I) bearing moieties -AM having solid support binding groups or precursors thereof are coupled to a solid support, for instance by reacting the compound of formula (I) with the solid support, with a spacer group on the solid support, or with reagents to introduce a spacer group and coupling the spacer group to the solid support. There are many possible compounds suitable for use in toning spacer groups. The spacer needs to be inert and not damaging to the target material in the context of the complete solid-supported reagent since, for instance, polymer modification may alter the properties of the solid support. The spacer needs to conserve the electron

withdrawing groups giving -AM'- an appropriate substituent constant as defined for soluble -AM polymer constructs. It is particularly important to avoid groups susceptible to nucleophilic attack. The selection of the spacer will also depend on the support selected and the properties defined above. The group on the sulphonyl activating moiety -AM for covalent bonding to the solid support will be capable of reacting with a reactive group on the solid support or spacer and will be chosen depending on the nature of the chosen solid support and any spacer group.

For instance, solid supports bearing aldehyde groups are reacted with an amino group on the compound of formula (I) in the presence of a non-nucleophilic base such as lithium isopropylamide so as to form a Schiff's base, followed by reduction of the double bond, for instance using an appropriate borohydride.

Suitable precursors of the compounds of formula (I) are readily available or can be produced by conventional techniques. Suitable solid support materials are also readily available; those which do not have groups capable of linking the precursor of the compound of formula (I) may readily be modified by conventional techniques in order to introduce the necessary functionalities, for instance siliconised glass (prepared as described by Mohr and Pommerening (1985) Affinity Chromatography, Practical and Theoretical Aspects, Marcel Dekker, Inc, New York and Basel) is activated using glutaraldehyde [Weston & Avrameas (1991) Biochemical and Biophysical Research Communications, 45, 1574-1580].

The compounds of formula (I) and solid supports bearing groups of formula (I') are readily hydrolysed and extremely labile and are preferably used when freshly prepared or, after preparation, are stored in air-tight containers such as glass ampoules prior to use.

<u>POLYMERS OF FORMULA (II)</u>

The polymers of formula (II) used in the present invention are all based on known and readily available polymers which generally already contain at least one group G, reactive with the group X of the compound of formula (I). As polymers are almost inevitably mixtures, the value of g will be an average for the material as a whole but is preferably nearly or, more preferably, exactly integral, preferably with a high degree of homogeneity. For polymers which have two or more reactive groups G it may be necessary or desirable to prevent reaction at a proportion of such groups by blocking them with a capping group C. The number of capping groups will also be an average for the material as a whole and is preferably nearly or, more preferably, exactly integral, or is zero when no capping groups are required. The polymers, reactive groups G and capping groups C will be further described below.

The polymers that can be used are selected from the following which are all readily available to those skilled in the art:

1. Homo- and heteropolymers, i.e. polymeric substances with repeating identical or non-identical subunits (homopolymers and heteropolymers respectively) such as:

(i) Polyalkylene compounds; for example

a), polyalkylene oxides and glycols and their derivatives such as poly(oxymethylene), polyethyleneglycols and oxides and methoxypolyethyleneglycols and related homopolymers, such as polymethylethyleneglycol, polyhydroxypropyleneglycol, polypropyleneglycols and oxides, polymethylpropyleneglycol, and polyhydroxypropyleneoxide, which may be straight-chain or branched polymers (for example straight-chain polypropyleneglycols and branched-chain polypropyleneglycols) and derivatives of the above including ethers, for instance of polyethyleneglycol or polypropyleneglycol such as the monomethyl ethers, monocetyl ethers, mono-n-butyl ethers, mono-t-butylethers and monooleyl ethers, esters of polyalkyleneglycols with carboxylic acids such as the monobutyl esters and monostearyl esters, and dehydration condensation products of the polyalkyleneglycols with amines such as propylamine and stearyl amine;
b) polyvinyl compounds such as poly(vinylpyrrolidone), polyvinyl alcohol, poly(vinyl acetate) and the copolymer poly(vinyl acetate-co-vinyl alcohol),poly(vinyloxazolidone),poly(vinylmethyloxazolidone) and poly(vinyl methyl ether);
c) polyacrylic compounds such as poly (acrylic acid)s, poly(methacrylic acid)s, polyhydroxyethyl-methacrylates, their amides such as poly(acrylamide) and poly(methacrylamide) and N-substituted derivatives of the amides such as poly(N,N-dimethylacrylamide), poly(N-isopropylacrylamide), poly(N-acetamidoacrylamide) and poly(N-acetamidomethacrylamide;
d) polyionic compounds such as poly(ethyleneimine), poly(ethylsulphonic acid), poly(silicic acid), poly(styrenesulphonic acid), poly(vinyl amine), poly(2-vinylpyridine) and its N-alkyl derivatives, poly-(4-vinylpyridine) and its N-alkyl derivatives, poly-(vinylsulphuric acid), poly(vinyl alcohol-co-vinyl sulphuric acid), poly(diallyldimethylammonium chloride), poly[(dimethylimino) trimethylene(dimethylimino)hexa-methylene dibromide)], poly(ethylenephosphonic acid), poly(maleic acid), poly(2-methacryloyloxyethane-1-sulfonic

acid), poly(3-methacryloyloxypropane-1-sulfonic acid), poly(4-vinylbenzoic acid); poly(4-vinylbenzyl-trimethylammonium), poly[3-(vinyloxy)propane-1-sulphonic acid)], poly(4-vinylphenol), (poly[p-hydroxystyrene]), poly(4-vinylphenyl sulphuric acid), poly(2-vinylpiperidine), poly(4-vinylpiperidine); poly(N-vinylsuccinamidic acid) and ionizable synthetic copolymers;

e) polyalkylene polyols such as polyoxyethylated glycerol, polyoxyethylated sorbitol (e.g polysorbates and polyoxyethylated glucose; and

f) non-ionic surfactants such as polyoxyethylenealkylphenols (including Tritons), polyoxyethylenemercaptans, polyoxyethylenealkylamines and polyoxyethylenealkylamides;

(ii) Polyamino acids or synthetic polypeptides such as, for example, polymers of D-glutamic acid and D-lysine, polylysine, polyalanine, polyglutamic acid, polyaspartic acid and polyproline;

(iii) Polysaccharides, crosslinked products thereof and polysaccharide containing material including branched or unbranched polysaccharides, comprising saccharide monomers such as glucose, mannose, galactose, fucose, fructose, xylose, arabinose, glucuronic acid, sialic acid (neuraminic acid), galacturonic acid, mannuronic acid, D-glucosamine and galactosamine, which may be homopolysaccharides or heteropolysaccarides, such as

a) dextran and dextran derivatives including dextran sulphate, p-aminoethyl cross-linked dextran, and carboxymethyl dextran;

b) cellulose and cellulose derivatives including methyl cellulose and carboxymethyl cellulose;

c) starches (amylopectin and amylose) and dextrins derived from starch, hydroxyethyl starches, agarose, ficoll or its carboxy methyl derivatives;

d) glycosaminoglycan chains of proteoglycans such as hyaluronic acid, chondroitin sulphates, dermatan sulphate, heparin, heparin fragments, heparin oligosaccharides, heparan sulphate and keratan sulphate;

e) carbohydrate-containing side chains of glycoproteins and glycolipids such as gangliosides, globosides and sulphatides;

f) polymers of sugar alcohols such as polysorbitol and polymannitol; and

g) other polysaccharides such as glycogen, glucans (e.g. laminaran), glycosaminoglycans, polysaccharide sidechains of glycoproteins or glycolipids, algal polysaccharides (e.g. alginic acid or polymannuronic acid and sulphated polysaccharides such as carrageenan and agar), pectins, plant gums, seed mucilages such as guaran, bacterial and fungal polysaccharides (e.g. xanthans, gellan, alginate, soleroglucan, schizophyllan, curdlan and pullulan);

(iv) other organic polymers including polymers and copolymners of, for instance, amines, olefins, esters, acetal, polyamides, carbonates, ethers, phenylene sulphides, silicones, urea formaldehyde condensation products, phenol formaldehyde condensation products, urethanes, melamine formaldehydes, epoxy resins, acrylic resins, allyl resins, polyacrylic acid and carbomers; and

(v) inorganic polymers especially those inorganic polymers containing organic moieties, for example, silicates.

2. Block copolymers, i.e. copolymers formed by combining appropriate blocks of the above polymers and include for instance block co-polymers of small alkoxymonomers, e.g. polyethylene/polypropyleneglycol (over 30 polyoxyethylene-polyoxypropylene copolymers are available on the market); block copolymers of ethylene and maleic anhydride; block copolymers of polyalkylene glycols and polyvinylpyrrolidone or polyvinyl alcohol; block copolymers of polyoxyethylene and polyoxy-propylene (Pluronics); block copolymers of the ethers, esters or dehydration condensation products of polymers of ethylene glycol and propylene glycol; and block copolymers of acrylamide and acrylic acid.

Typically the polymer will be water soluble. The polymer is preferably of very low toxicity in vivo and in vitro, since many applications involve administration to man or animals and/or exposure to cells in cell culture systems. The polymer length is not restricted in this invention, since it will depend on the principal use of the product (e.g. short lengths may be best for coupling two proteins together and longer polymers for improving plasma half life). With each application optimum polymer length must be selected either empirically or by reference to the desired application. The polymer's own inherent hydrophobicity is also important in some settings and will have to be evaluated on a case-by-case basis.

The reactive groups G are terminal hydroxyl groups. Depending upon the selected polymer such groups G may already be available for reaction or it may be necessary to introduce groups G by conventional techniques in a preliminary step. Generally groups G will be groups bonded to carbon atoms of the polymer. The groups G must be capable of reacting with the group X of the compounds of formula (I). The groups G will be selected such that they ultimately

permit formation of the desired non-immunogenic, non-antigenic, non-toxic, non-biodegradable direct covalent link between the polymer and target. Preferred groups G are primary (-$CH_2OH$) or secondary (-CHOH) alcoholic hydroxyls for instance primary hydroxyls as found at the termini of polyalkylene glycols such as in PEG. Such hydroxyls react with sulphonyl halides and other activating compounds of formula (I) to form activating sulphonate ester moieties.

The use of capping groups C is an important feature of the reaction since this embodiment of the invention has special advantages discussed further below. The function of the capping groups C is to protect one or more reactive groups G of the polymer molecule from nucleophilic attack while leaving the activated polymer product susceptible to nucleophilic attack only at the carbon or equivalent atom adjacent to the groups -AM. C therefore must lack the properties of -AM and should preferably be an inert group with respect to reactivity with other molecules and should be of low toxicity. Suitable groups C are well known to those skilled in the art as are techniques for introducing such groups. A preferred group C is methyl.

Production of the polymers for use in the process of the invention may be achieved by conventional techniques although most materials will be obtained commercially, possibly then being modified to introduce the desired reactive groups G and/or capping groups C as mentioned above.

A preferred polymer for use in accordance with the present invention is a PEG polymer HO-$(CH_2CH_20)_x$-H where x is large enough for the molecular weight to be in 150(x=3), 194(x=4), 238(x=5), 282(x=6) or above, for instance about 500, 1000, 2000, 3000, 4000, 5000, 6000 or 10000 which has two reactive groups G, i.e. the hydroxyl groups. The size of the polymer actually used will be selected according to the desired properties of the end product.

Another preferred polymer is MPEG (i.e. the polymer MeO-$(CH_2CH_20)_x$-H which has one capping group C, i.e. the methyl group and one reactive group G, i.e. the hydroxyl group) where x is 3, 4, 5 or 6 or is large enough to provide molecular weights of, for instance, about 500, 1000, 2000, 3000, 4000, 5000, 6000 or 10000. Branched PEGs can also be used.

Since conventional polymerization techniques generally produce materials which are mixtures, in certain cases some purification may be required before the polymer is used in the process of the invention, for example, some preparations of MPEG are significantly contaminated with PEG. This would lead to the production of biactivated PEG and crosslinking on subsequent use and needs to be avoided for many applications. Such contaminated material can be identified on the basis of its size distribution (polymerization occurs at both ends of the molecule for PEG hence the contaminant has circa twice the molecular weight). Size fractionation should therefore be used to remove this PEG from MPEG preparations prior to activation: gel permeation chromatography and other appropriate methods may be used. For example, the divalent PEG can be separated from the bulk MPEG using vesicle chromatography [Selisko et al (1993) J. Chromatogr. **641**, 71-79).

TARGET MATERIALS

Suitable target materials to which polymer can be attached in accordance with the present invention are all materials having biological activity which are useful in, for instance diagnosis or therapy and which are all well-known to those skilled in the art. They all contain at least one reactive group (hereinafter referred to as groups "N") containing an atom which is capable of mounting a nucleophilic attack on the carbon or equivalent atom of the polymer adjacent to the group AM. Examples of the reactive group include primary and secondary amino groups, thiol groups and aromatic hydroxy groups.

More specifically, potential targets include proteins, peptides, amino acids and their derivatives such as: antibodies and fragments thereof; cytokines and derivatives or fragments thereof, for example, the interleukins (IL) and especially the IL-1, IL-2, IL-3, IL-4, IL-5, IL-6, IL-7, IL-8, IL-9, IL-10 and IL-11 subtypes thereof; colony stimulating factors, for example granulocyte-macrophage colony stimulating factor, granulocyte-colony stimulating factor (alpha and beta forms) and macrophage-colony stimulating factor (also known as CSF-1); haemopoietins, for example erythropoietin, haemopoietin-alpha and kit-ligand (also known as stem cell factor or Steel factor); interferons (IFNS), for example IFNalpha, IFNbeta and IFNgamma; growth factors and bifunctional growth modulators, for example epidermal growth factor, platelet derived growth factor, transforming growth factor (alpha and beta forms), amphiregulin, somatomedin-C, bone growth factor, fibroblast growth factors, insulin-like growth factors, heparin binding growth factors and tumour growth factors; differentiation factors and the like, for example macrophage differentiating factor, differentiation inducing factor (DIF) and leukaemia inhibitory factor; activating factors, for example platelet activating factor and macrophage activation factor; coagulation factors such as fibrinolytic/anticoagulant agents including heparin and proteases and their pro-factors, for example clotting factors VII, VIII, IX, X, XI and XII, antithrombin III, protein C, protein S, streptokinase, urokinase, prourokinase, tissue plasminogen activator, fibrinogen and hirudin; peptide hormones, for example insulin and calcitonin; enzymes such as superoxide dismutase, glucocerebrosidase, asparaginase and adenosine deaminase; vaccines, for example hepatitis-B vaccine, malaria vaccine, melanoma vaccine and HIV-1 vaccine; transcription factors and transcriptional modulators; carbohydrates, glycosoaminoglycans, glycoproteins and polysaccharides; lipids, for example phosphatidyl-ethanolamine, phosphatidylserine and derivatives thereof; sphingosine; steroids such as cholesterol and derivatives thereof; nucleotides, nucleosides, heterocyclic bases, DNA, RNA, synthetic and non-synthetic oligonucleotides including those with nuclease resistant backbones; vitamins; antibiotics; bacteristatic and bactericidal agents; antifungal, anthelminthic and other agents effective against infective agents including unicellular pathogens; small effector molecules such as noradrenalin, alpha adrenergic receptor ligands, dopamine receptor ligands, histamine receptor ligands, GABA/bentodiazepine receptor ligands, serotonin receptor ligands, leukotrienes and tri-iodothyronine; cytotoxic agents such as doxorubicin, methotrexate and derivatives thereof.

The target molecules may also be part of larger multi-molecular structures. These include cells or parts thereof, for instance erythrocytes, erythrocyte "ghosts" and leukocytes, liposomes such as multilamellar vesicles and unilamellar vesicles, micelles and micelle-like structures, and aggregates, microemulsions, coacervates, emulsions, suspensions of the foregoing.

It will be appreciated that when the target molecules are part of such structures there will generally be many target molecules in each structure; treatment according to the invention will therefore produce a structure bearing many polymer moieties. Where the polymers are bi- or multivalent, reaction with a multimolecular target may result in intermolecular cross-linking by the polymer between molecules of the same target structure and/or between molecules of different target structures as well as intramolecular bonding of the polymer to more than one position on the same molecule of a target structure.

It is evident from the inventors' investigation that much of the loss of biological activity frequently observed with prior polymer coupling methods is due to inappropriate coupling reactions (i.e. reactions taking place at sites which disable the target's biological activity), coupling conditions and/or contaminating toxic material reducing responses in bioassays. The present process provides a means of generating adducts of polymer and target with highly conserved biological activity for the majority of molecules, or even enhanced biological activity. In the event of unusually substantial loss of biological activity, a search must be made using fractionated material with different degrees of substitution and appropriate mapping (e.g. peptide mapping) to identify the group or groups N the modification of which by polymer is responsible for lost activity. The situation can then be remedied either by modifying the group N to abrogate coupling of polymer at that site or by shielding N with an appropriate non-covalent bond (examples include hydrogen bonding of base pairs for DNA and protein/protein binding like receptor ligand interactions). Two alternative strategies are available depending on the nature of the critical N group, in particular whether there are significant differences in the susceptibility of the group compared to the other such groups (either being more or less susceptible) to coupling to the polymer. If the group N is less susceptible the coupling conditions (usually the coupling ratio) can be altered so that the critical group N is rarely modified. If it is more susceptible, a two step procedure where short polymer molecules are first linked to more accessible (susceptible) sites (i.e. including the group(s) N) then long polymer molecules are linked to the remaining sites may prove acceptable, the outcome depending on the nature of the problem at the N site with respect to biological activity.

Targets lacking a reactive group may be modified so as to create one or more reactive groups; this is within the ability of those skilled in the art and can be achieved by well-known techniques.

Some target structures (e.g. RNA and single stranded DNA) pose special problems because they provide too many "N" groups to which the polymer would attach in a standard reaction. There the groups N may be temporarily protected by involvement in an appropriate conformation precluding the nucleophilic attack, as for example in the hydrogen bonding associated with base pairing of DNA (see below).

An embodiment of the present process involves site-specific modification of DNA, RNA and synthetic oligonucleotide targets (or of any molecule containing an amino or other reactive group which can participate in interactions such as hydrogen bonding with another molecule or compound) by precluding the nucleophilic attack on the activated polymer species by reactive groups on the target. The bases adenine (A), cytosine (C) and guanine (G) [but not uracil (U) or thymine (T)] provide suitable targets in DNA, RNA and synthetic oligonucleotides for modification with polymer moieties according to the invention and thus these are special targets with the problem that there are too many available reactive groups to which the polymer can be attached. As shown below, single stranded DNA is rapidly and very heavily modified by activated PEG (TMPEG).

By using various restriction fragment DNA cleavage sites as a model system, the present inventors have shown that selected bases A, C or G can be modified by the expedient of leaving short stretches (e.g. 2-4 bases) single stranded. Adenine bases appear to be the most susceptible to much modification. Blunt ended double stranded DNA is not readily coupled under the conditions described, indicating that hydrogen bonding between base pairs is sufficient to preclude interaction of the amino groups of A, C and G bases with the activated polymers.

Site-specific DNA modification by polymer can be achieved by the expedient of including one or more A, C or G bases in a short single stranded section of DNA by appropriate restriction enzyme digestion or by hybridising oligonucleotides of dissimilar lengths with the DNA to protect bases which are not to be modified or by exploiting the natural strand asymmetry of polymerase chain reaction products which have a one base-pair overhang, or by exploiting localised regions of single strandedness achieved by natural or artificial localised melting of the double helix.

THE PROCESS

The process of the present invention relies upon conducting steps (a) and (b) in accordance with certain constraints as defined above. In particular, step (a) must be conducted using dry polymer, dry solvents and with water being excluded from the reaction vessel. Dryness of the polymer may be determined by benzene distillation as described below. If the polymer is not sufficiently dry, interfering side reactions occur making it difficult to obtain economical amounts of the final adduct. Likewise, wet solvents and ingress of water during the reaction will also cause interfering side reactions. Suitably dry solvents are obtained using molecular sieves (0.3nm) as described below. Exclusion of water from the reaction vessel can be achieved by conventional techniques.

Benzene distillation

The principal exploited here is that the water-benzene azeotrope has a boiling point of 65°C and that after water has been removed from a sample, the distilling temperature rises to that of pure benzene (i.e 79 to 80°C). In addition, the water-benzene azeotrope is cloudy and benzene is clear so that the change from cloudy to clear distillate confirms that drying is complete. If a suitably dry sample is heated in benzene the distillation temperature will be that of pure benzene and the distillate will be clear from the first.

0.3 nm Molecular sieves

Solvents may be dried by any conventional method provided that they are dried to the extent obtainable by use of 0.3 nm molecular sieves. Thus, for instance, when 0.3 nm molecular sieves are used an amount of molecular sieve is added to and left in contact with the solvent for several hours (preferably about 10 hours or more) and at about room temperature (ca 20°C), the amount of molecular sieves used suitably represents a twenty fold excess over the recommended amount to adsorb the anticipated water content in the solvent, according to the manufacturer's instructions, for instance about 100g of molecular sieve per litre of substantially dry solvent.

It is also a requirement that the solvent should be inert to the reagents and the activated polymer. Suitable inert solvents include halogenated alkanes such as dichloromethane and other organic solvents with low hydrophilicity and low hygroscopicity; such solvents are well known to those skilled in the art. Halogenated alkanes, especially dichloromethane, are preferred.

The activated polymer is preferably used directly following its production in accordance with step (a) as this affords the minimum opportunity for degradation leading to formation of other reactive species which will interfere with subsequent reactions. If the product is to be stored prior to use, for instance when formed in bulk for use in smaller quantities at a later date, it must be stored so as to avoid hydrolysis, i.e. in the presence of a more hygroscopic desiccant. Sulphonyl halides are highly hygroscopic and will dry many common desiccants such as silica gel and thus become hydrolysed. The preferred desiccant for drying and storage of the activated polymer is phosphorus pentoxide.

The reaction of the activated polymer with the target material in step (b) takes place in a non-denaturing medium; for many biological materials this will of necessity be an aqueous medium. There are also many target materials which are stable under non-aqueous conditions and for these it is quite acceptable to conduct step (b) in organic solvents which are inert to the reagents. Ultimately the choice of reaction medium will be dependant on the stability of the target material and final adduct in that medium. Selection of pH, salt concentration, protein concentration and other requirements for stability will be determined on a case-by-case basis. Those skilled in the art will have no difficulty with this. The same applies in relation to determination of non-denaturing temperatures.

When the process of the invention involves the use of a solid support coupled to the activated polymer, the reactions involved in coupling the activating moiety to the solid support will also be conducted under dry conditions, i.e. dry reagents as determined by benzene distillation and dry solvents as determined using molecular sieves of 0.3 nm, will be employed whenever sulphonyl halide groups are present.

It is preferred that the reaction of step (a) is conducted in the presence of a base such as a tertiary amine, preferred bases include those which are easily removed by washing, such as pyridine, and volatile bases which may be removed by evaporation. At present the preferred base is pyridine. However certain bases, especially pyridine, may react deleteriously with the target material and these should be removed prior to step (b). This may be achieved by conventional means.

It is preferred that the reagents for step (a) are mixed at reduced temperature, preferably at 0°C for instance by chilling with ice and that the reaction mixture is then allowed to warn to ambient temperature, for instance about 20°C.

These preferred features may be adopted in any combination but most preferably all of these preferred features are adopted.

When the polymer of formula (II) has more than one group G and/or one or more capping groups C, it has

been found that contamination with related products of formula (II,)

$$(C)_{c'} POL-G_{g'} \qquad (II')$$

wherein C, POL and G are as defined above and c' and g' are zero or positive numbers such that c'+g' = c+g but g' is different to g, can lead to undesirable inhibition of the intended reaction and formation of unwanted by-products which therefore complicates the purification and recovery of the eventual adduct. In the case where the polymer of formula (III) is a polyalkylene oxide with one capping group (eg MPEG), c is 1 and g is 1. Contaminants of formula (II') in which c' is 2 and g' is 0 will be unreactive and thus not detrimental but contaminants in which c' is 0 and g' is 2 will cause cross-linking of target materials; preferably such contaminants are avoided in production of the compounds of formula (II) or are removed prior to use of the compound in step (a) of the present process. When the polymer of formula (II) is a material in which c' + g' > 2 there are many possible contaminants; again those wherein g' is 0 are unreactive and of little concern whereas those wherein g' > 1 and g' > g, especially those wherein g' > g, cause unwanted side reactions and are preferably removed from the reagent before use.

Whilst the reaction of step (b) will proceed in the presence of large amounts of such contaminants, it is preferred that the amount of all compounds of formula (II') present in the compound of formula (II) is less than 35%, for instance less than 25%, more preferably less than 20%, for instance, 15, 10 or 5% and yet more preferably below 1% by weight based on the total weight of the compound of formula (II) and all compounds of formula (II'). This may be achieved by purifying the starting materials used in forming the polymer, controlling the polymerisation and subsequent process steps required to form the compound of formula (II) and/or by purifying the compound of formula (II) before use in the process of the invention.

In a preferred aspect of the invention the compound of formula (II) is MPEG in which POL is a divalent residue $-0-(CH_2CH_20)_x-$ (for instance the residue of PEG 5000), c is 1 and C is methyl and g is 1 and G is hydroxyl. This material is produced by polymerisation from methoxyethanol and ethylene glycol and tends to contain a large proportion of material where polymerisation has initiated from ethylene glycol rather than the methoxyethanol such that chain extension occurs in both directions and a PEG product of approximately twice the molecular weight of the MPEG is present as a major contaminant; this may readily be removed by gel permeation chromatography at an appropriate molecular weight cut-off.

Purity can be checked by nmr and chromatography. Contamination by materials where g' > g is particularly to be avoided when 1:1 target:polymer adducts are to be produced, since the presence of such contaminants will lead to the production of aggregated materials.

When the reaction of target and activated polymer is to be performed at a solid:liquid interface, the activated polymer may, in one variant of the process, be linked to a solid support after reacting a compound of formula (I) having a suitable linking group with the polymer. The linking reaction may be effected as described above in connection with the production of compounds of formula (I) linked to a solid support. Thus, for instance, a tetrafluoro-4-amino-azobenzene-4'-sulphonyl derivative of the polymer is reacted with a solid support bearing aldehyde groups (for instance siliconised glass treated with glutaraldehyde) in the presence of a non-nucleophilic base, such as lithium isopropylamide, followed by borohydride reduction of the potentially labile double bond.

It is particularly preferred that the process of the invention is conducted using tresyl chloride as the compound of formula (I) and MPEG, for instance MPEG 5000, as the polymer of formula (II). For MPEG of molecular weight up to 5000 or 6000, purity can be checked by nmr but for higher molecular weight material the methyl signal is swamped and chromatographic techniques must be adopted. These are well known to those skilled in the art.

In this preferred aspect, the product of formula (III) is TMPEG. Ideally this is used immediately but, if not, storage of TMPEG is critical and activity is not well maintained unless it is properly stored. When desiccated over phosphorus pentoxide and stored at 4°C, activity is maintained for at least 1 year. Degradation of TMPEG (either during production or on storage) is detectable on the basis of a change in the FPLC profiles of exposed peptide targets (see Example 1). It produces less substitution of the target at a given molar ratio (a loss of resolution and right shift of the elution profile accompanies the former if FPLC loads are not corrected for the amount of PEG loaded). Substantial aging of TMPEG prepared as previously described is documented in Figure 8 in WO90/04606. Since degradation produces coproduct, degraded TMPEG preparations are also more acidic.

PEG itself is known to oxidise on storage to a brown discoloured product. The TMPEG preparation process removes the antioxidants present in commercially supplied PEG. Oxidation has been observed in several stored TMPEG preparations and it is recommended that, if prolonged storage is anticipated, an appropriate antioxidant should be included. This new method for preparing and storing the TMPEG has significant advantages over previously described methods in terms of the activity, reproducibility between preparations and stability of individual preparations.

In an especially preferred aspect the present invention provides a process comprising the steps of

(a) reacting dry MPEG (as determined by benzene distillation) containing less than 10% by weight of PEG (as

determined by nmr and/or chromatography) with tresyl chloride in dry dichloromethane (as determined using 0.3 nm molecular sieves) in the presence of pyridine in a reaction vessel from which water is excluded, so as to form TMPEG, recovering the TMPEG so formed and either storing it over phosphorus pentoxide or using it directly in step (b),

(b) reacting the TMPEG so formed, optionally after storage, with a target material such as erythropoietin (EPO) in aqueous medium and about 20°C and

(c) recovering the Target:MPEG adduct (eg EPO:MPEG) so formed.

More preferably the molar ratio of tresyl chloride to MPEG is 2:1 or more, most preferably about 2.5:1.

The process of the present invention may be supplemented by one or more separation and/or purification steps at any stage as necessary or desirable. Separation and purification may be achieved by appropriate techniques well known in the art selected having regard to the need to maintain the activity of the reagents and products and to avoid use of toxic or interfering materials. In some cases, for instance where the polymer is a polyalkylene glycol, aqueous biphasic polyalkylene glycol solutions may be used to effect separation and/or purification of the activated polymer and/or polymer:target adduct.

THE PRODUCTS

The present invention further provides polymer:target adducts obtainable by the process of the invention other than the known polymer:target adducts having polymer and target moieties directly linked by stable, non biodegradable covalent bonds which are described in the references discussed above.

The invention also extends to such products for use in therapeutic and diagnostic methods of treatment of the human or animal body and to the use of such products in the manufacture of medicaments for use in therapeutic and diagnostic methods of treatment of the human or animal body and to pharmaceutical compositions comprising products of the invention together with pharmaceutically acceptable diluents or carriers.

Many of the prior methods of coupling polymers to targets are unsuitable for coupling more than one target molecule to each polymer molecule since most methods use polymers having only one group capable of reacting with the target. In contrast, the process of the present invention enables the production of constructs such as the following which form particular embodiments of the present invention:

1. Liposomes bearing linear polymeric linking groups bound to the surface of the liposome at one terminal of the polymer and bearing a receptor ligand, receptor molecule, antibody, antigen or other molecule of therapeutic or diagnostic interest at the other terminal.
2. Constructs where a plurality of identical target molecules are linked to a single polymer moiety; for instance a linear polymer having two reactive termini is linked to two target molecules to enhance the biological activity thereof or a branched polymer having three or more reactive termini or a polymer having reactive pendant groups is linked to three or more target molecules.
3. Constructs as in 2. above wherein the target molecules are different; such constructs may be intended to have synergistic effect when the target molecules interact with each other or with other substrates.
4. Constructs wherein the polymer moiety is linked to a target molecule at two or more positions either by reaction of both termini of a linear polymer with different sites on the target molecule or by reaction of pendant reactive groups, or the termini of a branched polymer, at two, three or more sites on the target molecule.

The products of the present invention preferably comprise any one of the polymer materials set out above and any one or more of the target materials set out above. Particularly preferred products of the invention are adducts of a polyalkylene glycol, especially polyethylene glycol and polypropylene glycol with the former being most preferred, with any one or more of the target materials set out above, with erythropoietin (EPO) being particularly preferred.

The production of adducts wherein two or more different target species are coupled to a multivalent polymer presents special difficulties which can be overcome by the process of the present invention. For this situation an appropriate procedure for producing a product comprising two different targets involves the following steps:

1. A first target, Target (A), is exposed to activated polymer (AM-Polymer-AM) with the latter in gross excess such that essentially only 1:1 Target(A):polymer adducts are formed and 2:1 or higher Target(A):polymer adduct formation is minimised. This can be followed by FPLC or other appropriate methods to separate and, if necessary, purify the adduct, provided that this is sufficiently rapid to avoid hydrolysis of the remaining activating moieties. If the target has many derivatisable groups, it may be necessary to fine-tune the coupling ratio, reaction time and/or pH, so that 2:1 or lower Target(A):polymer adducts are not formed or, if formed are discarded. Alternatively a mixture of monovalent and multivalent activated polymer species may be used in a ratio selected such that it is statistically

likely that only one of the polymer molecules attached to any target molecule will be the multivalent form (targets which are by chance only modified with the univalent form will be wasted).

2. Either:

a) the Target(A):polymer adduct is first separated from the AM-polymer-AM then reacted with the second target, Target(B), preferably at equimolar ratio or, if this reacts too slowly, with the adduct in excess; or
b) the reaction product of step 1 is reacted with Target (B), preferably at a molar ratio of 1:2 with respect to the amount of activating moieties remaining in order to reduce the formation of Target(B):polymer:Target(B) constructs. This is much simpler than (a) but more wasteful in terms of Target(B) which is therefore selected as the cheaper of the targets.

After a sufficient reaction time (derived empirically), the excess -AM is neutralised with a monovalent neutralising agent e.g. with glycine, and the products are purified to discriminate the combinatorial possibilities. This may be possible on the basis of molecular weight if target(A) and target(B) differ significantly in size or <u>via</u> differential labelling of the targets. Alternately, since the major contaminant will be Target(B):polymer:Target(B) at this stage, an affinity purification method, with affinity ligand to Target(A), should enrich for hybrids. Similar measures may be adopted to construct more complex adducts.

A particular advantage of the present invention is that the process enables the coupling of polymer moieties to oligonucleotides and nucleic acids (DNA and RNA) at defined positions. EP-A-O 292 128 (Sager 1988) deals with "improved DNA probes" and uses PEG, amongst other molecules, to link DNA to reporter groups for hybridisation. However the PEG is linked to the 5'-phosphate of the DNA and there is no indication of a method which would link the PEG without leaving a coupling moiety. This ability to modify DNA, RNA and synthetic oligonucleotides with polymer will influence the solubility and alter biodistribution and has applications in both the <u>in vitro</u> and therapeutic or diagnostic (<u>in vivo</u>) use of nucleotides.

Further improvements offered by the invention include the ability to maximise the biological activity retention and/or to increase the activity of the target molecule, minimise the toxicity of the product, minimise the reaction time at physiological pH, reduce contamination of the product and improve the stability of the activated polymer.

The invention will be illustrated with reference to the accompanying figures of the drawings in which:

Fig.1 shows the results of separations of MPEG 5000 obtained commercially.
Fig.2 is a plot of age of TMPEG used and % of gm-CSF left unmodified by reaction with aged TMPEG produced according to the invention.
Fig.3 is a plot of the relative activities of PEGylated gm-CSF prepared by the process of the invention and by a previously described process.
Fig.4 plots the activity of EPO and PEG-EPO.
Fig.5 is a gel showing DNA fragments variously treated with TMPEG, MPEG or buffer.
Fig.6 shows log concentrations of gm-CSF and various PEG-gmCSF adducts in blood and tissue of mice.
Fig.7 shows the results of fractionation of gm-CSF and PEG-gm-CSF before and after ageing.
Fig.8 shows FPLC profiles for EPO PEGylated with various molar ratios of TMPEG:lysine.
Fig.9 shows biological activity of unmodified and PEGylated EPO.

The present invention will now be illustrated by the following Examples which are not intended to limit the scope of the invention in any way:

<u>EXAMPLES</u>

<u>Examples 1a and b and Comparative Examples 1a and b</u>

(a) <u>Purification of MPEG</u>

MPEG-5000 (Union Carbide) was subjected to permeation gel chromatography on Superose 12 (Fig. 1a). The material has a clearly biphasic size distribution with an estimated 22.9% high molecular weight contaminant (9280Da) and a low molecular weight component (5460Da). The good agreement between proportions of the two peak areas and the NMR estimate of PEG versus MPEG in the same preparation suggest that all the PEG is accounted for by the high molecular weight species. To confirm this and to devise an appropriate purification procedure, vesicle chromatography [Ehwald (1991)] was used. Analytical and preparative scale preparations gave different efficiencies of separation. Analytical scale separation was performed using vesicular packing material P1(5) equilibrated in 0.05M $NaH_2PO_4$ buffer pH 5.5 (column bed volume 22 ml). The MPEG preparation (Union Carbide Mw 5000), 0.5ml of a

20mg/ml solution in PBS was loaded onto the column. The column was eluted with PBS using a flow rate of 0.08 ml/min and 0.5 ml fractions were collected. The elution profile is shown in Fig.1b; circa 8% of the material was eluted in the excluded fraction (high molecular weight) and 92% in the permeable fraction (low molecular weight fraction). In preparative scale (using 6.5L bed volume and 15g/500ml MPEG in 0.01M $NaH_2PO_4$ pH 5.5 and an elution rate of 20.5 ml/min) the excluded fraction (PEG) was 11.2 % (Fig.1c). Rechromatography on Superose 12 of the two pooled fractions (shaded and hatched areas of Fig.1c) taken from the preparative scale fractionation on Superose 12 was used to determine the extent to which the two materials were separated (Fig. 1d, squares - permeable fraction, crosses - excluded fraction). NMR analysis of the permeable fraction gave an estimated ratio of PEG:MPEG of 11:89 mole:mole, which is in good agreement with the area under the curve estimate of the residual high molecular weight contaminant in the permeable fraction on the elution profile on Superose 12 (Fig.1d, squares). NMR analysis could not be performed on the excluded fraction because of its much higher molecular weight. Further rounds of vesicle chromatography, at analytical scale, gave a comparable reduction in the high molecular weight material (the permeable fraction yielded a smaller "excluded" peak on rechromatography, circa 4% in the high molecular weight excluded fraction, using a second round of vesicle chromatography, Fig. 1e).

(b) Preparation of TMPEG

Tresyl chloride was used when freshly prepared or after storage in airtight ampoules. MPEG-5000 (Mr 5000, 18g, 3.5 mmol) was dried with benzene (B.P. 79-80°C) by distillation of the water-benzene azeotrope and then the benzene. The water-free MPEG was then immediately dissolved in dichloromethane (45 ml, ANALAR, previously dried over molecular sieve 3Å, 100 g per litre of solvent, overnight at room temperature). Activation of MPEG-5000 with tresyl chloride was carried out at a molar ratio of 2.5:1 (tresyl chloride to available hydroxyl groups in MPEG). The MPEG/dichloromethane mixture was then cooled on ice (to circa 3°C) and pyridine (1 ml, 12.4 mmol) followed by tresyl chloride (1 ml, 9 mmol), both precooled on ice were added dropwise with constant stirring using a magnetic stirrer. The reaction was allowed to continue (for 2 hr) at room temperature with constant stirring before the removal of dichloromethane by evaporation under reduced pressure. The solid obtained (TMPEG) was redissolved in methanol-hydrochloric acid mixture (1000:0.3 v/v) and allowed to precipitate overnight at reduced temperature (-20°C). The precipitate was recovered by centrifugation (at 1100 x g, for 10 minutes) at reduced temperature (0°C) and the supernatant was spectrophotometrically checked for pyridine content (λmax 255nm). This procedure was repeated until no pyridine could be detected, usually 12 to 15 washes. Finally, the pyridine-free precipitate was washed in methanol only, preferably at least twice, and then the methanol was removed by evaporation under reduced pressure and the TMPEG was finally dried by freezing in liquid nitrogen and lyophilising under reduced pressure. The hydroxyl content of the white solid as determined by $^1H$ nmr obtained was undetectable (4.56ppm). Therefore, approximately 100 % of hydroxyl groups in the MPEG were transformed into tresyl esters.

The TMPEG so produced was desiccated over phosphorus pentoxide and stored at 4°C.

(c) Coupling to GM-CSF

The activity of TMPEG samples was monitored by exposure of the samples to gm-CSF at molar ratios of 305:1 (Example 1a and Comparative Example 1a) or 75:1 (Example 1b and Comparative Example 1b) in a standard coupling reaction and estimating the percent of the preparation remaining unmodified after the reaction as shown in Fig.2: the relative activities of TMPEG preparations obtained as described above (Examples 1a and b) and of TMPEG made by a prior art process (Comparative Examples 1a and b) is plotted against the storage period. TMPEG made by the process of the invention and used at 305:1 (squares), throughout the observation period, gave <10% residual unmodified gm-CSF. The equivalent result when using the same material at 75:1 (diamonds) was circa 20% unmodified material. With both preparations there was no evidence of a trend of worsening activity with storage. In contrast preparations made from MPEG as obtained from the manufacturer by a previously described method (WO90/04606, Example 1) (triangles; downwards triangles 305:1 and upwards triangles 75:1) showed: (1) lower mean activity (reflected in higher mean residual unmodified material); (2) a very broad scatter of reactivities; (3) 3 samples completely inactive at 75:1 and 2 inactive at 305:1 after only 5-12 weeks of storage. At 305:1 the process of the invention consistently produced 92-95% modification, irrespective of the age of the TMPEG and using a coupling ratio of 75:1, circa 80% of the gm-CSF preparation was modified.

The process described above (Example 1) has been repeated on a number of occasions with similar results.

The gm-CSF PEGylated using the process of Example 1 showed no significant loss of its growth stimulating activity as assessed by granulocyte-macrophage colony assay (Fig.3a) or by thymidine uptake (Fig. 3b). Dose response analyses are a relatively insensitive means for demonstrating subtle differences in bioactivity and are also difficult to analyse after pooling because of the high contribution to errors of the variation in the response level between experiments. A better approach is to perform a correlation analysis, plotting the response obtained with equivalent amounts

of the modified and unmodified materials on an XY plot. Identical activity gives datum points randomly distributed about the line representing equal activity (the dashed lines). Reduced activity reveals itself as a curvilinear departure from the equal activity line which converges towards the line at the extremities (if sufficiently broad a dose response range is examined such that the plateau of maximum stimulation is reached). In contrast, the presence of inhibitory material, with or without loss of intrinsic activity, produces a departure, usually without convergence to the upper extremity. In the latter case (i.e. where there is no loss of intrinsic activity) failure to observe divergence at the lower extremity indicates that the inhibitor is masking activity only at relatively high inhibitor levels; divergence throughout the plot indicates either an inhibitor with powerful effect at low dilution or combined inhibition and loss of activity.

Two assays for the growth stimulating activity of GM-CSF prepared by the methods of Example 1 (▣ in Fig.3) and Comparative Example 1 (□ in Fig.3) show that the process of the invention gives better conservation of biological activity. Colony counts or 3H-thymidine uptake (normalised with respect to the maximum per experiment to avoid a contribution of between-experiment differences to the correlation) were plotted for comparable amounts of PEG-GM-CSF and GM-CSF over the dose range spanning the upper and lower asymptotes of the dose response curve (0.1 to 8 ng/ml for colony assays and 0.0006 to 1.0ng/ml for thymidine uptake assays). Datum points are means of colony counts in triplicate dishes for three independent experiments (Fig. 3a) or of two independent thymidine uptake experiments (Fig 3b). The results in the thymidine uptake demonstrate no significant loss of activity with the process of Example 1 and loss of activity without significant inhibitory material for the process of Comparative Example 1 (TMPEG in equivalent doses was not inhibitory in this assay). The results for colony stimulating activity again show no significant loss of activity with process of the invention but indicate an additional inhibitory activity for this assay when the old method was used possibly accompanied by loss of bioactivity.

Example 2

a) Preparation of ditresyl PEG (TPEGT)

PEG-6000 (10.5 g, 1.75 mmol) was dissolved in benzene (30 ml) and dried as in Example 1 above. The dry PEG-6000 was then immediately dissolved in 25 ml of dichloromethane (dried as in Example 1), cooled on ice and pyridine (1 ml, 2.4 mmol) followed by tresyl chloride (1 ml, 9 mmol) were added dropwise. The mixture was left to react (two hours at room temperature) and the dichloromethane was evaporated as in Example 1. The solid was resuspended in methanol:hydrochloric acid and was treated as above.

b) Coupling to GM-CSF

To test that this material was bivalent and could couple target(s) at both ends of the PEG chain, this TPEGT was used to aggregate GM-CSF. $^{125}$I-GM-CSF (1.4nmol/ml) was reacted with TPEGT (160mg/ml) for 2h at room temperature. The resulting aggregates were estimated by FPLC and contained 95% of the total GM-CSF. Thus the bivalent AM-polymer-AM construct is demonstrated to be functional.

Example 3

Example 2 was repeated using TMPEG prepared from a sample of MPEG containing 8% PEG as contaminant, and thus containing about 8% TPEGT. When used to aggregate GM-CSF, this material (160 mg/ml) produced an aggregate estimated by FPLC to contain only 20% of the total GM-CSF. This demonstrates that the higher proportion of conjugates seen in Example 2 was related to the additional TPEGT and not merely due to non-specific aggregation. It should be noted that using a prior art process, the present inventors have shown [Malik at al (1992) Experimental Hematology, **20**, 1028, fig. 4] that both the non-specific aggregated gm-CSF in unPEGylated reactions and aggregates generated in PEGylation reactions (which include cross-linked products due to TPEGT) have an extremely high specific activity, as assessed by thymidine incorporation, with respect to monomeric gm-CSF). Thus it is anticipated that the bivalent 2-polymer construct generated in Examples 2 and 3 would have a higher specific activity than monomeric unmodified gm-CSF.

Example 4 and Comparative Examples 2,3 and 4

To document the better conservation of biological activity, direct comparisons were made with three of the widely used current methods, using erythropoietin as the target peptide. It is an important prerequisite in experiments of this type to perform preliminary experiments (using at least two independent assays of bioactivity and some measure of the degree of modification) to select the optimum coupling ratio and duration of coupling reaction for maximum retention of biological activity. It is important to record the degree of modification since this may vary between the methods

being analysed and only materials substituted to approximately the same extent should be compared for retention of activity.

Serial dilutions of TMPEG produced as in Example 1 were mixed with the sue amount of erythropoietin and the resultant PEG-erythropoietin adducts were assessed first by FPLC for the degree of modification and also in the thymidine uptake assay [Malik et al., (1992) Experimental Hematology, 20: 1028] for a preliminary estimate of activity. Based on the latter a smaller range of activities were analysed in colony assays using the methyl cellulose assay system of Ash et al., Blood, 58:309-316 (1981), Knusli, C. et al. (1992) Br. J. Haematol. 82(4): 654.

Commercially available activated PEGs (Sigma Limited) were used to PEGylate recombinant human erythropoietin (Cilag Ltd.) by the cyanuric chloride (Comparative Example 2), succinimidyl succinate (Comparative Example 3) and phenylchloroformate (MPEG-p-nitrophenylcarbonate) methods (Comparative Example 4). TMPEG, prepared as described above, or the alternate activated PEGs, were incubated (for 2 h) with erythropoietin (100 μg/ml total protein, i.e. carrier plus erythropoietin) in coupling buffer (PBS) at room temperature in a rotary mixer, at an activated-PEG:lysine molar ratio of 75:1. Conditions were standardised so that differences in pH and duration of the coupling reaction could not account for differences in the residual bioactivity. Representative reactions at the same molar ratios were checked by FPLC and found to be ≥85% modified. Modified material was assayed in methylcellulose cultures of normal human bone marrow progenitor cells essentially by the method of Ash et al (1981) Blood, 58 309-316, but substituting recombinant human GM-CSF for leucocyte conditioned medium. Erythroid bursts were scored microscopically on encoded, encrypted dishes on day 14-16 of culture. The results for the optimised TMPEG-derived adducts and for products of the other coupling reactions having the same coupling ratio are shown in Fig.4 as means of triplicate culture dishes except in Panel A where the results are means ± SEM for 3 independent experiments. Fig 4a shows the excellent conservation of biological activity achieved with EPO PEGylated with the TMPEG (filled circles) compared with unmodified EPO (open circles). Datum points are superimposed except at 4U/ml (33.6 ng/ml) erythropoietin. Similar effects observed in CFUgm assays were found to be due to a modest inhibitory effect of TMPEG in the assay system.

Fig. 4b shows a complete loss of detectable activity when the cyanuric chloride method (filled circles) was used compared with PEG-EPO prepared according to Example 1 (open circles) and a high dose inhibition of endogenously stimulated erythroid colonies. This indicates either the presence of a profoundly inhibitory substance in the reaction mixture or loss of all intrinsic erythropoietic activity in conjunction with some inhibitory material. Lack of even a slight increment in colony formation at low doses and the shallow dose related inhibition of endogenously stimulated colonies at high concentrations favours the latter interpretation. The FPLC profile for this material also differed somewhat from that obtained with the other three activated PEG preparations in that there was a higher proportion of material eluting with the void volume, indicating the presence of more aggregates. This suggests that crosslinking of target molecules may be occurring (as has previously been observed for this agent [Leonard (1984)]).

Fig. 4c shows a somewhat different picture for PEG-EPO prepared by the succinimidyl succinate method (filled circles), with detectable activity only at 4U/ml (33.6ng/ml) compared with PEG-EPO according to the invention (open circles). This type of shift in the dose-response curve is less likely to be due to inhibitor and probably represents over an order of magnitude loss in bioactivity. This is somewhat surprising because this method is claimed to have good conservation of biological activity [Katre (1987), Zalipsky (1992)]. However such claims have been made on the basis of short term assays. In the assay used here the erythropoietin needs to be available throughout the 14-16 day culture period. The cells in these cultures are also a rich source of esterases and the PEG-erythropoietin made by this method contains a potentially labile bond (see Table 1). In this and some related active ester methods, removal of the PEG leaves the molecule modified as follows:- Target-$NH_2$ forms

$$\text{Target-NH-CO-CH}_2\text{-(CH}_2)_n\text{-CO-PEG}$$

then esterase cleavage forms

$$\text{Target-NH-CO-CH}_2\text{-(CH}_2)_n\text{-COOH.}$$

This process might well influence bioactivity adversely since there is a charge conversion from a lysine amino group to a carboxyl group at the previously PEGylated site.

Fig. 4d shows PEG-erythropoietin made using monomethoxy PEG-p-nitrophenylcarbonate (filled circles) compared with unmodified (sham-treated) EPO (open circles). Here there are increments in colony number at low doses similar to that seen with the tresyl chloride activated PEG, but the high-dose inhibition of colony formation is much more evident, indicating the presence of inhibitory material.

Comparative Example 5

An attempt was made to make PEG-erythropoietin using the carbonyidlimidazole method under comparable

conditions to those used above, but on this time scale and coupling ratio the degree of modification achieved was not significant.

Example 5

A solution (50µl) of amphotericin (Fungizone) in distilled water (20 mg/ml) was mixed with HEPES buffer (450µl, pH 9.0) containing TMPEG produced according to Example 1 (60 mg/ml). The mixture was then incubated at room temperature for 24 h. As a control, amphotericin was similarly treated but with MPEG substituted for TMPEG. To demonstrate the covalent linkage of the TMPEG to the amphotericin, the partition coefficient of amphotericin in both samples was measured as follows: 100µl samples were mixed with 1 ml of top and 1 ml of bottom phases of a biphasic system containing 5% dextran T-500, 5% PEG-6000, 0.15 M sodium chloride and 0.01 M sodium phosphate pH 6.8. After shaking for 1 min. the system was left to settle for 20 min. and then 500µl from the top and bottom phases were removed to quantify amphotericin (by measuring the absorbance at 330 nm). The % material in the top (PEG-rich) phase was: a) control 4.9 ± 0.2%; b) MPEG control 4.1 ± 0.4%; c) TMPEG (preparation 1) 92.8 ± 0.1%; d) TMPEG (preparation 2) 92.7 ± 0.2%.

Example 6

As an example of creation of an affinity ligand PEG-WGA (polyethyleneglycol modified wheat germ agglutinin) was constructed in exactly the same manner as used in Example 1 and used to bind rightaide-out vesicles from human erythrocyte membranes thus altering their partition in a PEG-dextran aqueous two-phase system. Rightside-out vesicles exposed to phosphate buffered saline prior to partitioning showed the following distribution: 17.6 ± 1.2% to the top phase, 48.7 ± 9.0% to the interface and 33.6 ± 8.6% to the bottom phase (mean ± SEM, n=3). Rightside-out vesicles exposed to the PEG-WGA ligand prior to partioning showed the following distribution: 30.1 ± 1.2% to the top phase, 32.7 ± 1.4% to the interface and 7.2 ± 0.6% to the bottom phase. This change in partitioning can be exploited to remove rightside-out vesicles from a mixture with inside-out vesicles thus obtaining a pure preparation of inside-out vesicles from human erythrocyte membranes.

Example 7

Aliquots of human erythrocytes (50µl containing 0.95 x $10^7$ cells) were added to one of the following reagents (500µl):

a) sodium phosphate buffer (0.05M, pH 7.5) containing sodium chloride (0.125M) (PBS).
b) TMPEG freshly prepared as in Example 1 in PBS (36 mg/ml, equivalent to 1.9 ng/cell).
c) TMPEG (36 mg/ml) inactivated with lysine (Sigma Ltd. 4mg/ml) at 2.5:1 molar lysine:TMPEG for 2h at room temperature.

After 30 min incubation at 37°C cells were pelleted down and resuspended in PEG-rich top phase (1 ml) so that their PEGylation could be monitored in a PEG-dextran phase system. Cells incubated in PBS partitioned with 26.2 ± 11.8% (mean ± SD) cells in the top phase. With TMPEG exposure of cells this increased to 72.1 ± 10.7%, whereas with prior inactivation of TMPEG to prevent coupling to cells there was only 32.7 ± 9.3% in the top phase, indicating that covalent attachment of the PEG to the erythrocytes was responsible for the change in partitioning behaviour. The erythrocytes remained intact in the TMPEG-treated and control experiments indicating that the method does not disrupt the cell membrane.

Example 8 and Comparative Example 6

Liposomes were reacted with TMPEG prepared by the process of WO90/04384 (Comparative Example 6) and the process of Example 1 (Example 8). The success of the PEGylation was followed in a PEG-dextran phase system by quantitating the liposome content of the top (PEG-rich) and bottom (dextran-rich) phases and at the interface. Because the TMPEG prepared by the process of Example 1 was known to be more active, only half the molar ratio was used. Despite this, a much higher degree of substitution of the liposome was achieved. This greater reactivity has the advantage that the liposomal preparation does not have to be exposed to so much activated PEG (the level of the latter is limited by its effect on liposomal stability and/or aggregation). 100 nm diameter unilamellar vesicles of bovine spinal cord phosphatidylserine (PS) were prepared by extruding lipid dispersions of PS (10 mg/ml in HEPES buffer pH9), spiked with $^3$H-dipalmitoylphosphatidylcholine through 100 nm polycarbonate filters ten times using an extruder device (Lipex Biomembranes, Canada). Vesicles (300 µl) were incubated with TMPEG (39 mg, molar ratio TMPEG:PS at outer

surface 4:1 or 19.5 mg, molar ratio 2:1) in HEPES buffer (60 µl) for 18 hrs at room temperature. As a control MPEG was substituted for TMPEG. To demonstrate the covalent attachment of PEG to the outer surface of the vesicles the distribution of the vesicle in a two-phase system was measured as follows: samples (20 µl) were mixed with top phase (1 ml) and bottom phase (1 ml) of a biphasic system containing dextran T-500 (5%) and PEG 6000 (5%), sodium chloride (0.15M) and sodium phosphate buffer (0.01M) prepared at 25°C. After shaking for 1 min, triplicate samples (50 µl) were removed for total counts. After the phases had separated at 25°C for 20 min triplicate samples were taken from the top phase (50 µl) and the bottom phase (20 µl) for scintillation counting. Vesicle partitionings, calculated as the percentage of added vesicles that were present in the top(T) and bottom(B) phases and at the interface (100-T-B) were as shown in Table 2.

Table 2

| TMPEG sample | TMPEG:PS Molar ratio | Partitioning (%) (mean of triplicate partitions) | | |
|---|---|---|---|---|
| | | Top | Interface | Bottom |
| Old Method | 4:1 | 6.3±0.6 | 25.3±4.0 | 68.4±3.7 |
| Control MPEG | | 5.5±0.3 | 9.1±4.9 | 85.4±4.8 |
| New Method | 2:1 | 71.6±1.2 | 11.1±1.2 | 17.3±0.5 |
| Control MPEG | | 4.9±0.2 | 1.6±1.6 | 104.3±7.3 |
| | | | | |

The increase in interface partitioning with associated decrease in bottom phase partitioning with the old method indicated some PEGylation. However with the activated PEG made by the new method, even at the lower molar ratio there is extensive partitioning to the top phase indicating the highest degree of PEGylation of the two samples.

To increase the PEGylation with the rather unreactive old method sample, the molar ratio was increased to 20:1, using a final concentration of 35% w/w TMPEG. This caused the vesicles to aggregate. Controls with MPEG also showed aggregation. Addition of 75µl of the reaction mixture to 2.2 ml of N-tris-(hydroxymethyl)methyl-2-amino-ethane-sulphonic acid (TES) buffer [Fisher et al (1991) Cell and Model Membrane Interactions p.47 Plenum Press, New York] pH 7.4 gave solutions with $OD_{650}$ of 0.26 whereas untreated vesicles in control buffer had $OD_{650}$ of 0.01, indicating that the aggregation observed with the TMPEG and MPEG was not completely reversible. It is well established that PEG solutions can produce fusion and/or permeability increases [Tilcock & Fisher (1982) Biochimica et Biophysica Acta, **688** 645-652, Aldwinkle et al., (1992) ibid, 689: 548]

Example 9

(a) 1µg of the supercoiled plasmid pBR322 was digested with 100 units of the restriction enzyme Rsa I for two hours at 37°C, in low salt buffer consisting of 10mM Tris HCl pH 7.5, 10mM $MgCl_2$, 1 mM dithiothreitol **(DTT).** This yields four blunt ended fragments per molecule of pBR322. Protein was removed by phenol/chloroform extraction. After ethanol precipitation the DNA was resuspended in a small volume of tris-EDTA **(TE)** pH 7.6 and the DNA was end labelled with [γ32P] dATP using a BCL kit as per the manufacturer's instructions. Removal of the free [$^{32}$P] dATP was achieved using Sephadex G-50 columns. The DNA was then split into 6 aliquots. Three of these aliquots were made single stranded by boiling and then exposed to either TMPEG or MPEG, at a concentration of 400mg/ml in coupling buffer (consisting of 0.005M sodium phosphate 0.125M sodium chloride pH 7.5) or to coupling buffer only, at a volume ratio of 150 µl:150µl), for 20 minutes. Three other aliquots, which were allowed to remain double stranded, were incubated with either TMPEG, MPEG or coupling buffer only, under identical conditions. After incubation, all the samples were again made mingle stranded by boiling. The samples were examined in a 2% alkaline agarose gel, (50mM NaOH, 1mM EDTA pH 8.0) using alkaline loading buffer. This was a 6x stock consisting of 0.3M NaOH, 6mM EDTA pH 8.0, 18% Ficoll 400, and 0.15% bromocresol green. The samples were run overnight at 50v. The dried gel (Fig. 5) was analysed by autoradiography, the film was developed in an automated Fuji X-ray film developer. Lanes 1-6 from left to right are: 1-3 DNA exposed while double stranded; 4-6 DNA exposed while single stranded; 1&4 coupling buffer; 2&5 MPEG; 3&6 TMPEG. Only single stranded DNA exposed to TMPEG showed a mobility shift demonstrating coupling of PEG to the DNA.

(b) Eco RI digested pBR322 containing a single cleavage mite yielding one double stranded fragment with a 3' recessed 4bp cut and the following single stranded overhangs:-

5'NC......AATTCN3'
3'NGTTAA......GN5'.

Of three aliquots, one was treated with a reaction mixture consisting of TMPEG (400 mg/ml) in coupling buffer (50 ml), DNA (circa 0.1 µg in 50 ul of tris EDTA). The coupling buffer (pH 7.5) was a solution of sodium chloride (0.125M) and sodium phosphate (0.05M). The reaction was conducted at room temperature for 20 min. A second aliquot was similarly teated with TMPEG (40 mg/ml) in coupling buffer and the third was treated with coupling buffer alone.

(c) Bam HI digested pBR322 containing a mingle cleavage site yielding one double stranded fragment with a 3' recessed 4bp cut and the following single stranded overhangs:-

5'NG......GATCCN3'
3'NCCTAG......GN5'.

This material was divided into three aliquots which were treated with two concentrations of TMPEG and coupling buffer as in (b).

To detect the extent of PEG-modification the staggered ended DNA was end-labelled with $^{32}P$ and then partitioned in a PEG-phosphate phase system to determine the partition coefficient (K), results being shown in Table 3.

Table 3

| | No PEG | TMPEG 40mg/ml | TMPEG 400mg/ml |
|---|---|---|---|
| DNA digest | EcoR1 50µg/ml | ECoR1 50µg/ml | ECoR1 50µg/ml |
| PEG-phase (%)* | 41.7 | 58.4 | 96.5 |
| $PO_4$-phase (%)* | 58.3 | 41.5 | 3.5 |
| log(K) | -0.145 | -0.148 | 1.44 |
| DNA digest | BamH1 50µg/ml | BamH1 50µg/ml | BamH1 50µg/ml |
| PEG-phase (%)* | 41.7 | 51.0 | 69.4 |
| $PO_4$-phase (%)* | 58.3 | 49.0 | 30.6 |
| log(K) | -0.145 | -0.017 | 0.356 |

* Yields corrected for differential recovery from the phases via recovery controls.

Up to maturation there is known to be a linear relationship between the number of PEG molecules attached per target molecule and log(K). The DNA cleaved with EcoRI (with two free amino groups supplied by two adenine bases) is more readily modified than the DNA cleaved with BamHI (with three free amino groups supplied by adenine, guanine and cytosine).

Example 10

BALB/c, male, 6-8 week-old mice, weighing approximately 20g, were pooled into groups of three per condition, with *adlib* access to food and water. PEG-modified GM-CSF samples were prepared as in Example 1, using the concentrations of GM-CSF and TM-PEG indicated in Table 4a. The reaction products were evaluated by FPLC and the proportions of individual species were estimated by fitting summated Gaussian curves to the FPLC profiles and estimating the area under the curve corresponding to each individual peak and expressing this as a % of the total area for the whole FPLC profile (Table 4b). The inventors have previously established that the individual peaks seen at low TMPEG concentrations (peaks 0, 1, 2 and 3) correspond to GM-CSF, $PEG_1$-GM-CSF, $PEG_2$-GM-CSF, $PEG_3$-GM-CSF, respectively. At high TMPEG concentrations the peaks are broader and less well resolved thus fitting of individual Gaussian curves over all regions of the FPLC profile may not be possible. Thus peak 3 and the peaks for more highly substituted GM-CSF (potentially $PEG_{4-7}$-GM-CSF) have been pooled. The peak eluting with the void volume peak is well

resolved from the $PEG_{4-7}$-GM-CSF and presumably represents aggregates, the area of this peak was used to estimate the proportion of aggregated material (Table 4b).

The different PEGylated samples and unmodified GM-CSF (Hoechst), all containing <4% [$^{125}$I]GM-CSF (Amersham, 1222 Ci/mmol) to serve as a tracer, were adminstrated at a dose of 0.0524µg in 100µl (3.45 x $10^{-12}$ mole; equivalent to 2.5µg/kg body weight) by subcutaneous injection. The amount of TMPEG concomitantly administered is also indicated in Table 4a (MPEG has been shown not to influence $t_{1/2}$, but does influence egress from the subcutaneous site). Up to eight 25µl tail vein blood samples were collected from each mouse using capillary tubes at various time points ranging between 10 minutes and 80 hours, to give a minimum of three samples per time point. Samples from the capillary tubes were washed immediately into 1ml of PBS containing 50 I.U. of heparin. Radioactivity was determined using a gamma counter (LKB 1270 Rackgamma) results being shown in Fig. 6a (open circles for untreated material, other symbols as indicated on the figure). Clearances for the materials of reactions A and B were clearly biphasic, presumably reflecting the large proportion of unmodified material remaining in these preparations (Table 4b). Half-lives were calculated using linear regression (log concentration versus time) for the slower clearing component if biphasic elimination was present (solid lines Figure 6a; dotted lines give the 95% confidence intervals). The $t_{1/2}$ values ±SE are given in Table 5.

With the exception of the product of reaction D, which is known to contain >50% of aggregates (the latter are known to clear slowly), $t_{1/2}$ for the other reactions (A-C) are similar. Although there may be some increment in $t_{1/2}$ between reactions A and B, surprisingly, there is no additional increment in $t_{1/2}$ betwen reactions B and C. This was not anticipated in view of the fact that reaction C produced a higher proportion of $PEG_2$-GM-CSF and adducts with higher PEGylation ratios. The difference in $t_{1/2}$ between reactions A and B is also not marked and, with the proviso that dissection of early and late components introduces more error into the $t_{1/2}$ estimates, it can be inferred that the addition of the first PEG molecule has the greatest impact on the clearance of GM-CSF. This is unexpected because the estimates for apparent molecular weight from FPLC were initially: GM-CSF 14.5kDa; $PEG_1$-GM-CSF 40.5kDa; $PEG_2$-GM-CSF 67.6 kDa; $PEG_3$-GM-CSF 112.9 kDa and after 3 or 4 independant estimates the values were: GM-CSF 12.5 ± 1 kDa; $PEG_1$-GM-CSF 41.5 ± 3.6 kDa; $PEG_2$-GM-CSF 75.2 ± 10.5 kDa; $PEG_3$-GM-CSF 138.5 ± 27.3 kDa. Thus, $PEG_1$-GM-CSF should not exceed the renal molecular weight threshold for excretion and the major increment in the $t_{1/2}$ would have been expected to be between $PEG_1$- and $PEG_2$-GM-CSF not between GM-CSF and $PEG_1$-GM-CSF. This result is also surprising in that the increment in apparent molecular weight is unexpectedly large for the addition of the first MPEG 5000 molecule (12.5 to 41.1 kDa). Other proteins (e.g. recombinant erythropoietin) have shown a surprisingly small increment in apparent molecular weight with the addition of one MPEG 5000 molecule (eg circa 1.4 kDa increment).

In order to establish the contributions to extended $t_{1/2}$ we examined the tissue distribution of material prepared in an identical manner to reaction C, using an identical dose. At each of the times indicated, one aminal was sacrificed and organs/tissue including kidney, spleen, liver, lung, heart and femur were recovered and radioactivity was measured as above. Figure 6b shows the results. The dotted and dashed lines indicate the blood levels of GM-CSF and PEG-GM-CSF respectively. The filled circles and open circles are the organ concentrations of PEG-GM-CSF and GM-CSF respectively. Comparison of organ:blood concentration ratios indicates that renal accumulation (the kidney is the only organ which achieves high organ:blood ratios and is the site which is known to catabolise the peptide to small fragments), is substantially reduced by PEGylation of the degree achieved in reaction C. This is somewhat surprising since circa 24% of the reaction C mixture represents $PEG_1$-GM-CSF (i.e. material below the renal threshold). It suggests that the size of PEG conjugates is not the only factor in reduction of renal clearance. Thus the degree of substitution desirable for individual targets will have to be assessed on a case-by-case basis.

Table 4(A)

| Coupling Ratios and Compositions for PEGylated GM-CSF Preparations | | | | |
|---|---|---|---|---|
| **PREPARATION** | **GM-CSF (µg/ml)** | **TMPEG (mg/ml)** | **GM-CSF (dose, µg)** | **TMPEG (dose, mg)** |
| Unmodified Control | 0.524 | 0 | 0.0524 | 0 |
| Reaction A | 0.524 | 6.46 | 0.0524 | 0.646 |
| Reaction B | 0.524 | 19.75 | 0.0524 | 1.975 |
| Reaction C | 0.524 | 197.5 | 0.0524 | 19.75 |
| Reaction D | 0.524 | 459.7 | 0.0524 | 45.97 |

Table 4(B)

| Percentage of GM-CSF in different fractions at various coupling reactions. | | | | | |
|---|---|---|---|---|---|
| | **Unmodified GM-CSF (Peak 0)** | **PEG-modified-GM-CSF** | | | |
| | | **Peak-1** | **Peak-2** | **Peak-3 & higher** | **Putative Aggregates** |
| Unmodified Control | 100 | 0.0 | 0.0 | 0.0 | 0.0 |
| Reaction A | 48.6 | 27.7 | 14.1 | 3.1 | 0.0 |
| Reaction B | 31.0 | 26.0 | 21.6 | 8.0 | 1.6 |
| Reaction C | 16.8 | 23.5 | 15.9 | 19.2 | 20.7 |
| Reaction D | 11.1 | 37.7* | | | 51.2 |

* Combined data for all PEG-GM-CSF Peaks (incompletely resolved)

Table 5

| Half-life of late clearing components in PEGylated (GM-CSF) preparations). | | | |
|---|---|---|---|
| | $t_{1/2}$ (hours) | $t_{1/2}$ + SE* | $t_{1/2}$ -SE* |
| Unmodified Control | 1.63 | 1.81 | 1.47 |
| Reaction A | 11.7 (7.2) | 14.0 (8.6) | 10.1 (6.2) |
| Reaction B | 18.1 (11.1) | 23.1 (14.2) | 14.8 (9.1) |
| Reaction C | 16.8 (10.3) | 17.3 (10.6) | 16.4 (10.1) |
| Reaction D | 25.1 (15.3) | 26.0 (15.9) | 24.2 (14.8) |

* calculated from the slope of the regression of log concentration versus time over portions of dose response curves (shown in fig.6(a)), ± the standard error of the slope.
Figures in parentheses indicate the fold increase over the control figure.

Example 11.

A direct comparison using a 2h. coupling period and a molar ratio of activated PEG:lysine of 305:1 gave the following results (assessing modification by phase partitioning as described by Delgado (1990)) is shown in Table 6.

Table 6

| PEGYLATION METHOD | PARTITION COEFFICIENT |
|---|---|
| Unmodified Control | 1.54±0.07 |
| The present invention (TMPEG) | 4.77*0.04 |
| Cyanuric Chloride | 9.0±0.74* |
| Carbonyldiimidazole | 2.06±0.14 |
| Phenylchloroformate | 4.92±0.02 |
| Succinimidyl succinate | 3.35±0.25 |

* Result compounded by the presence of large amounts of cross-linked products for which log K will not be a linear function of modification.

These results demonstrate that a fast reaction rate is achieved with the present invention so that there is less time for a target material to be damaged by the possibly hostile environment of the coupling reaction.

Example 12

After storage at 4°C, $^{125}$I-GM-CSF shows degradation with the appearance of label in low molecular weight material. This process is slowed by PEG modification. Fig.7 shows FPLC profiles of representative examples stored for 1 day (upper panels) and 33 days (lower panels). When supplied, $^{125}$I-GM-CSF has little or no low molecular weight labelled species. After storage for 1 day of an unmodified (open circles) preparation and a modified preparation (exposed to TMPEG at a molar ratio of 305:1 as described above, filled circles) there was a small amount of labelled low molecular weight material (peak 3) in both preparations. In both, the majority of the material (peak 1) is high molecular weight (the size distribution difference is commensurate with the PEGylation of the intact protein and the low molecular weight material probably represents free iodine). After storage the low molecular weight peak had increased substantially and a second peak (peak 2) had appeared between it and the peak of unmodified material (lower panel). There has been generation of intermediate and low molecular weight fragments in both samples. The former are commensurate in size with proteolytic cleavage products and the latter either with free iodine or very small fragments (i.e. < circa 1kDa). The relative amounts of $^{125}$I in the samples is listed in the Table 7. Pegylation reduces the loss of high molecular weight species.

Table 7

| | Peak | Before Storage | After 1 Month Storage |
|---|---|---|---|
| GM-CSF | 1 | 76.1 | 9.2 |
| | 2 | 0 | 20.6 |
| | 3 | 21.5 | 68.2 |
| PEG-CM-CSF | 1 | 81.1 | 45.8 |
| | 2 | 0 | 8.9 |
| | 3 | 12.4 | 36.3 |

Example 13

Dry stored TMPEG, prepared as in Example 1 above, derived from an MPEG preparation (Union Carbide UK) contaminated with circa 22% PEG (when estimated by NMR) was used in this Example. This contaminating PEG results (during the polymer activation process) in the formation of biactivated tresyl PEG which produces aggregates in the final product; the formation of aggregates can be reduced by reducing the level of contamination of the MPEG preparation by PEG. The erythropoietin (EPO) preparation used was a commercial preparation obtained from Cilag Ltd and the erythropoietin stock contained 3200 u/ml in PBS. This preparation contains 3200 units of EPO, and 2 mg/ml of human serum albumin. The coupling ratios were calculated on the basis of the total number of available lysine groups in the erythropoietin and carrier protein, and would need to be adjusted accordingly for pure erythropoietin preparations. The erythropoietin preparation was exposed to the stated molar ratios of TMPEG:lysine and was incubated at room temperature for two hours on a rotary mixer. The sham-reacted control was similarly exposed to diluent, (PBS). The resulting samples were analysed by FPLC the profiles being shown in Fig.8 panels A to F; the vertical axis in all cases is C.P.M.[$^{125}$I]-erythropoietin: A) Sham treated;B to F TMPEG:lysine at ratios B)9.375:1; C) 18.75:1; D) 37.5:1; E) 75:1; F) 305:1

Samples (200µl containing circa 10.5µg/ml total proteins) were analysed on a Pharmacia FPLC system with a Superose 12 HR 10/30 column previously equilibrated with PBS. The samples were loaded onto the column and then eluted with sterile PBS at a flow rate of 0.3ml per minute; 0.25ml fractions were collected. To monitor protein profiles, PEG-EPO was prepared using [$^{125}$I]-erythropoietin (Amersham) and fractions were analysed using a gamma counter (Nuclear Enterprises NE1600). To check the biological activity of a series of preparations made using the same TMPEG preparation, over a similar range of molar ratios of TMPEG:lysine, using unlabelled EPO (Cilag), serial dilutions of the unfractionated reaction mixture were exposed to the haemopoietic cell-line, TF-1, to induce thymidine uptake. Cultures were performed in microtitre plates containing 50 µl of cell suspension (5 x $10^4$ cells/well) in RPMI-1640 medium containing 5% foetal calf serum (FCS) in round bottom microwell plates (NUNC). To increase sensitivity of TF-1 cells to cytokine, they were deprived of their maintenance growth stimulus (GM-CSF at 50 units/ml) for 24 hours prior to assay. Each well contained either PEG-erythropoietin or unmodified erythropoietin, diluted in RMPI-1640. Cultures were incubated in a humidified atmosphere of 5% carbon dioxide in air at 37°C for 18 h. Cells were then exposed to 0.5µCi of [methyl-$^3$H]thymidine 25Ci/mmol. Amersham) in 50µl of culture medium and incubation was continued as above for 4 h. Cells were harvested onto glass filters (Titertek Flow labs) using a Dynateck multimesh 2000 harvester, washed with methanol and dried for 12 h at room temperature. [$^3$H]-thymidine incorporation was determined by scintillation counting (Beckman LS 5000 CE) using 5ml Filter Count (Packard) the results being expressed as D.P.M. of the test sample minus D.P.M of unstimulated cells; negative values therefore indicate inhibition of background thymidine uptake.

Fig.9a shows the induction of thymidine uptake by all preparations, both PEG-modified and unmodified. Fig. 9b shows the early portion of the dose-response curves for concentrations less than one unit/ml at expanded scale to facilitate comparison. All preparations produced stimulation of incorporation of thymidine, but there is a progressive contamination by inhibitory material, whose activity is most evident at 3 and 10 units/ml of erythropoietin. The inhibitor is related to the TMPEG:lysine molar ratio suggesting that either TMPEG itself or co-product is responsible (TMPEG does have an inhibitory effect in this assay system [Malik (1992)]. This inhibitory material was not evident to a similar degree in PEG-GM-CSF preparations, even those prepared with the same batch of TMPEG, and its nature has not yet been determined but may reflect altered sensitivity of the cell line to inhibition under different growth conditions. The similarity of the upward portion of the dose-response curves indicates that there is little inherent loss of biological activity for all the preparations studied.

<u>Example 14</u>

NMR analysis showed that a standard preparation of MPEG-5000 (kindly donated by Union Carbide) contained 23.5 mole % PEG and 76.5 mole % MPEG. Method: 1H-nmr spectra were acquired on a Brucker WN 259 spectrometer on polymer samples in DMSO-$d_6$ (50mg/ml). Integrations of the peaks at 4.56ppm (OH), 3.56 ppm ($CH_2CH_20$) and at 3.26 ppm ($OCH_3$) were made and used to calculate the PEG and MPEG contents (also the molecular mass of the sample which was 5200Da).

PEG contamination results from its production as a by-product during the polymerisation reaction for the manufacture of MPEG (hydroxide used to catalyse the reaction, initiates a side reaction yielding PEG). Since PEG has two termini at which polymerisation can take place, it should achieve a larger molecular weight than that of the MPEG. These data confirm that this side reaction takes place largely during the early phase of the reaction, since the size distribution of the PEG product is approximately double that of the MPEG product and there is no substantial' proportion of material spanning a range of intermediate sizes (as would be anticipated if the side reaction continued throughout the polymerisation).

To confirm that the contaminating PEG is, as anticipated, responsible for aggregate formation during the coupling reaction, a TMPEG preparation derived from the same MPEG preparation analysed above was fractionated on the expectation that it would contain circa 25.5% biactivated PEG. [Such fractionation requires prolonged exposure to an aqueous environment and resulted in some overall loss of activity (thus requiring appropriate sham-treatment of unfractionated controls) and is therefore not advisable as a step in the process of the invention, rather purification prior to activation is recommended]. The TMPEG was then exposed to $^{125}$I-gm-CSF under standard coupling conditions as described over a range of molar ratios as indicated in Table 8 (aggregate formation is more likely at higher molar ratios

and/or concentrations of polymer and target). Aggregates were identified by FPLC on Superose 12 (as labelled material eluting with the void volume) and PEG-modified GM-CSF species (as labelled material eluting faster than unmodified GM-CSF, but not as fast as the aggregated material). This identification has been confined independently by phase partitioning. The fractionation by vesicle chromatography separates material less prone to produce aggregates (permeable fraction) and more prone (excluded fraction) than the starting material respectively. This validates two points; first that the PEG becomes biactivated and second that the reduction of the contaminating PEG by size fractionation will reduce he amount of aggregates. Since the acceptable level of aggregates will vary from application to application a limit for contamination of MPEG by PEG will have to be selected on a case-by-case basis.

**Table 8**

**Reduction of PEG contamination reduces aggregate formation. Aggregates are expressed as % total PEG-modified material (aggregates + $PEG_n$-gm-CSF where n=1-7)**

**Table 8**

| | Unfractionated MPEG | Permeable Fraction | Excluded Fraction |
|---|---|---|---|
| PREP 1 (150:1 TMPEG:lysine) | | | |
| Aggregates | 21.6 | 6.1 | N.T. |
| PREP 2 (120:1) TMPEG:lysine) | | | |
| Aggregates | 17.9 | 0 | N.T. |
| PREP 3 (17:1 TMPEG:lysine) | | | |
| Aggregates | 0 | 0 | 13.3 |

**N.T. = Not Tested**

## Claims

1. A process for producing an adduct of a polymer and a target material which process comprises the steps of

(a) reacting tresyl chloride with a polymer of formula (II)

$$(C)_c\ POL - G_g \qquad (II)$$

wherein

POL is a polymer moiety of valency c+g,
C is a capping group and c is zero or a positive number and
G is a terminal hydroxyl group reactive with the compound of formula (I) and g is a positive number

so as to form

a sulphonate ester-activated polymer of formula (III)

$$(C)_c \text{ POL - (tresyl)}_g \quad \text{(III)}$$

wherein
C, POL, -AM, c and g are as defined above

(b) reacting the activated polymer of formula (III) with the target material and
(c) recovering the adduct of the polymer and the target material,
in which process:

(i) the polymer of formula (II) is dry as determined by benzene distillation, and
(ii) the reaction of the tresyl chloride with the polymer of formula (II) is conducted in an organic solvent which is inert to the reagents and to the product of formula (III) and is anhydrous as obtainable using molecular sieves of 0.3nm;
(iii) the reaction of the tresyl chloride with the polymer of formula (II) is conducted in a reaction vessel from which water is excluded;
(iv) the activated polymer of formula (III) so produced is recovered and either used directly in step (b) or stored, prior to use in step (b), in the presence of a desiccating agent more hygroscopic than the product of formula (III); and
v) the reaction of the activated polymer with the target material is conducted in a non-denaturing medium and non-denaturing temperature with respect to the target material.

**2.** A process according to claim 1 comprising, in step (b), reacting, in predetermined molar ratio and at predetermined concentrations, an activated polymer having two tresyl moieties with a first target material ($target^1$), so as to produce a sulphonate ester-activated 1:1 adduct of polymer and $target^1$, then reacting the 1:1 adduct with a second target material ($target^2$) so as to produce a $target^1$:polymer:$target^2$ adduct, wherein the first and second target materials may be the same or different, and then recovering the adduct in accordance with step (c).

**3.** A process according to claim 1 comprising reacting an activated polymer bearing a plurality of activating tresyl moieties with a first target material, wherein the proportion of the tresyl moieties on the activated polymer which react with the first target material is controlled such that in at least a proportion of the molecules of intermediate polymer:target adduct produced there remain unreacted tresyl moieties and wherein such untreated tresyl moieties are then reacted with one or more different further target materials so as to form materials polymer:$[target]_n$ wherein n is 2 or more and the first and further target entities are different; or the unreacted tresyl moieties are reacted with more of the first target material so as to form materials polymer:$[target]_n$ wherein n is 2 or more and the target entities are all the sane; or the unreacted tresyl moieties are reacted with further unreacted groups in the first target entity of the intermediate polymer:target adduct under conditions which hinder intermolecular reactions, such that the polymer moieties are bonded at two or more positions to the target entity.

**4.** A process according to claim 1 comprising, in step (b), reacting, in predetermined molar ratio and at predetermined concentrations, an activated polymer having only one tresyl moiety with a target material having more than one reactive group so as to form an adduct of the polymer and target having a preselected number of polymer molecules per molecule of target, or having a preselected proportion of the reactive groups reacted with and linked to polymer molecules, and then recovering the adduct in accordance with step (c).

**5.** A process according to claim 1 comprising, in step (b), reacting, in predetermined molar ratio and at predetermined concentrations, an activated polymer having two or more activating tresyl moieties with a target material having two or more reactive groups so as to produce a sulphonate ester-activated 1:1 adduct of polymer and target then changing the reaction conditions so as to hinder intermolecular reactions whilst permitting intramolecular reactions between activating tresyl moities on the polymer and reactive groups on the target so as to form adducts of polymer and target having two or more covalent bonds between the polymer and target moieties, and then recovering the adduct in accordance with step (c).

**6.** A process according to any preceding claim wherein the polymer is selected from polyalkylene compounds, polyvinyl compounds, polyacrylic compounds, polyionic compounds, polyalkylene polyols, non-ionic surfactants, polyamino acids, synthetic polypeptides, polysaccharides, crosslinked products thereof and polysaccharide containing material; other organic polymers and inorganic polymers and block copolymers thereof.

7. A process according to claim 6 wherein the polymer is selected from poly(oxymethylene), polyathyleneglycols and oxides, methoxypolysthyleneglycols, polymethylethyleneglycol, polyhydroxypropyleneglycol, polypropyleneglycols and oxides, polymethylpropyleneglycol, polyhydroxypropylentoxide, straight-chain and branched-chain polypropyleneglycols and derivatives thereof, polyethyleneglycol and polypropyleneglycol and the monomethyl ethers, monocetyl ethers, mono-n-butyl ethers, mono-t-butylethers and monooleyl ethers thereof, esters of polyalkyleneglycols with carboxylic acids and dehydration condensation products of the polyalkyleneglycols with amines and other polyalkylene oxides and glycols and derivatives thereof, poly (vinylpyrrolidone), polyvinyl alcohol, poly (vinyl acetate), the copolymer poly (vinyl acetate-co-vinyl alcohol), polyvinyloxazolidone, poly (vinylmethyloxazolidone and poly (vinyl methyl ether), poly(acrylic acid)s, poly(methacrylic acid)s, polyhydroxyethylmethacrylates, Poly(acrylamide and poly(methacrylamide) and other amides thereof, poly(N,N-dimethylacrylamide), poly(N-isopropylacrylamide), poly(N-acetamidoacrylamide) and poly(N-acetamidomethacrylamide, and other N-substituted derivatives of the amides, poly(ethyleneimine), poly(ethylsulphonic acid), poly(silicic acid), poly(styrenesulphonic acid), poly(vinyl amine), poly(2-vinylpyridine) and its N-alkyl derivatives, poly (4-vinylpyridine) and its N-alkyl derivatives, poly(vinylsulphuric acid), poly(vinyl alcohol-co-vinyl sulphuric acid), poly(diallyldimethylammonium chloride), poly((dimethylimino) trimethylene(dimethylimino)hexamethylene dibromide), poly(ethylenephosphonic acid), poly(maleic acid), poly(2-methacryloyloxyethane-1-sulfonic acid), poly-(3-methacryloyloxypropane-1-sulfonic acid), poly(4-vinylbenzoic acid), poly(4-vinylbenzyltrimethylammonium), poly[3-(vinyl-oxy)propane-1-sulphonic acid)], poly(4-vinylphenol)(poly[p-hydroxystyrene]), poly(4-vinylphenyl sulphuric acid), poly-(2-vinyl piperidine), poly(4-vinylpiperidine), poly(N-vinylsuccinamidic acid), polyoxyethylated glycerol, polyoxyethylated sorbitol, poly sorbates and polyoxyethylated glucose, polyoxyethylene-alkylphenols, polyoxyethylenamercaptans, polyoxyethylene- alkylamines, polyoxyethylene-alkylamides, polymers of D-glutamic acid and D-lysine, polylysine, polyalanine, polyglutamic acid, polyaspartic acid, polyproline, branched or unbranchad polysaccharides comprising saccharide monomers selected from glucose, mannose, galactose, fucose, fructose, xylose, arabinose, glucuronic acid, sialic acid (neuraminic acid), galacturonic acid, mannuronic acid, D-glucosamine and galactosamine, dextran, dextran sulphate, p-aminoethyl cross-linked dextran, carboxymethyl dextran and other duxtran derivatives, cellulose, methyl cellulose, carboxymethyl cellulose and other cellulose derivatives, starches (amylopectin and amylose) and dextrins derived from starch, hydroxyethyl starches, agarose, ficoll and its carboxy methyl derivatives, glycosaminoglycan chains of proteoglycans selected from hyaluronic acid, chondroitin sulphates, dermatan sulphate, heparin, heparin fragments, heparin oligosaccharides, heparan sulphate and keratan sulphate, carbohydrata-containing side chains of glycoproteins and glycolipids selected from gangliosides, globosides and sulphatides, polysorbitol, polymannitol and other polymers of sugar alcohols, polysaccharides selected from glycogen, glucans, laminaran and glycosaminoglycans, polysaccharide sidechains of glycoproteins and glycolipids, alginic acid, polymannuronic acid, carrageenan, agar and other sulphated polysaccharides, and other algal polysaccharides, pectins, plant gums, guaran and other seed mucilages, xanthans, gellan, alginate, scleroglucan, schizophyllan, curdlan, pullulan and other bacterial and fungal polysaccharides, polymers and copolymers of amines, olefins, esters, acetal, polyamides, carbonates, ethers and phenylene sulphides, silicones, urea formaldehyde condensation products, phenol formaldehyde condensation products, urethanes, melamine formaldehydes, epoxy resins, acrylic resins, allyl resins, polyacrylic acid and carbomars, silicates and other inorganic polymers containing organic moieties, block copolymers of polyethylene/ polypropylene-glycol, block copolymers of ethylene and maleic anhydride, block copolymers of polyalkylene glycols and polyvinylpyrrolidone or polyvinyl alcohol, block copolymers of polyoxyethylene and polyoxypropylene, block copolymers of the ethers, esters or dehydration condensation products of polymers of ethylene glycol and propylene glycol, and block copolymers of acrylamide and acrylic acid.

8. A process according to any preceding claim wherein the target is selected from proteins, antibodies and fragments thereof, , IL-1, IL-2, IL-3, IL-4, IL-5, IL-6, IL-7, IL-8, IL-9, IL-10, IL-11 and other interleukins and subtypes thereof, and other cytokines and derivatives or fragments thereof, granulocyte-macrophage colony stimulating factor, the alpha and beta forms of granulocyte-colony stimulating factor, macrophage-colony stimulating factor, and other colony stimulating factors, erythropoietin, haemopoietinalpha and kit-ligand and other haemopoietins, IFNalpha, IFNbeta and IFNgamma and other interferons, epidermal growth factor, platelet derived growth factor, transforming growth factor (alpha and beta forms), amphiregulin, somatomedin-C, bone growth factor, fibroblast growth factors, insulin-like growth factors, heparin binding growth factors, tumour growth factors and other growth factors and bifunctional growth modulators, macrophage differentiating factor, differentiation inducing factor (DIF), leukaemia inhibitory factor and other differentiation factors, platelet activating factor, macrophage activation factor, and other activating factors, heparin, proteases and their pro-factors, clotting factors VII, VIII, IX, X, XI and XII, antithrombin III, protein C, protein S, streptokinase, urokinase, prourokinase, tissue plasminogen activator, fibrinogen, hirudin, other fibrinolytic/anticoagulant agents and other coagulation factors, peptide hormones, enzymes, vaccines, transcription factors and transcriptional modulators, carbohydrates, glycosoaminoglycans, glycoproteins and polysaccharides, phosphatidylethanolamine and phosphatidylsermne and derivatives thereof, sphingosine, cholesterol

and other steroids and derivatives thereof, nucleotides, nucleosides, heterocyclic bases, DNA, RNA, synthetic and non-synthetic oligonucleotides, vitamins, antibiotics, bacteristatic and bactericidal, antifungal, anthelminthic agents, noradrenalin, alpha adrenergic receptor ligands, dopamine receptor ligands, histamine receptor ligands, GABA/benzodiazepine receptor ligands, serotonin receptor ligands, leukotrienes and tri-iodothyronine and other small effector molecules, doxorubicin, methotrexate and other cytotoxic agents and derivatives thereof.

**9.** A process according to any one of the preceding claims comprising the steps of

(a) reacting dry MPEG (as determined by benzene distillation) containing less than 10% by weight of PEG (as determined by nmr and/or chromatography) with tresyl chloride in dry dichloromethane (as determined using 0.3 nm molecular sieves) in the presence of pyridine in a reaction vessel from which water is excluded, so as to form TMPEG, recovering the TMPEG so formed and either storing it over phosphorus pentoxide or using it directly in step (b),
(b) reacting the TMPEG so formed, optionally after storage, with a target material such as erythropoietin (EPO) in aqueous medium and about 20°C and
(c) recovering the Target:MPEG adduct (eg EPO:MPEG) so formed.

**10.** Use of the product of any process defined in any preceding claim in the manufacture of a medicament for use in a therapeutic or diagnostic method of treatment of the human or animal body.

Fig.1a.

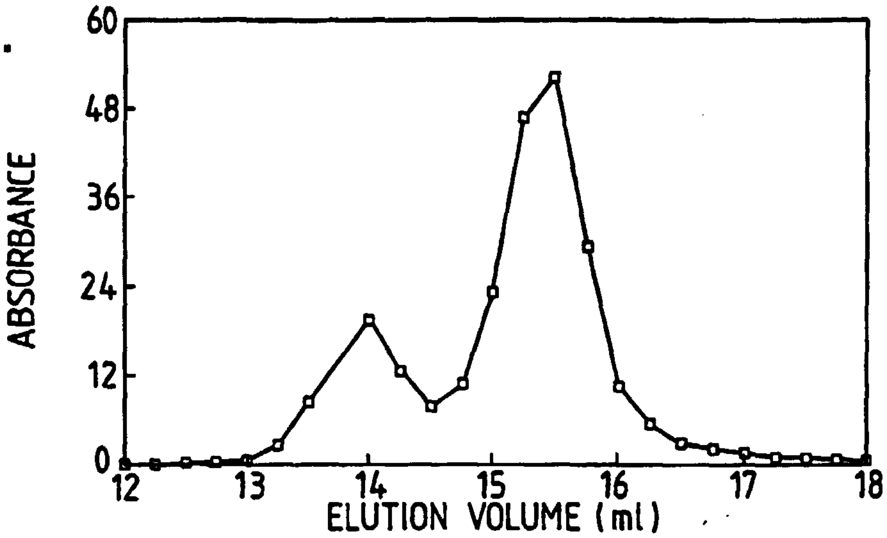


Fig.1b.

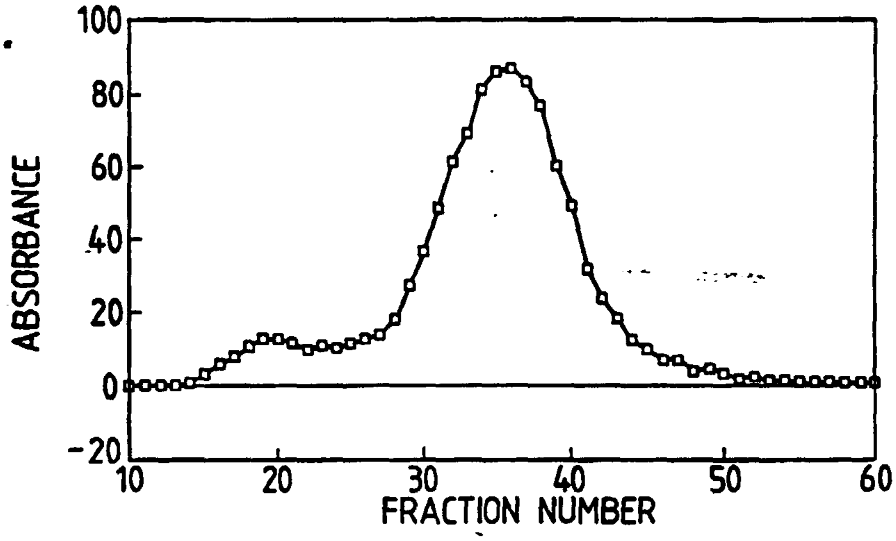


Fig.1c.

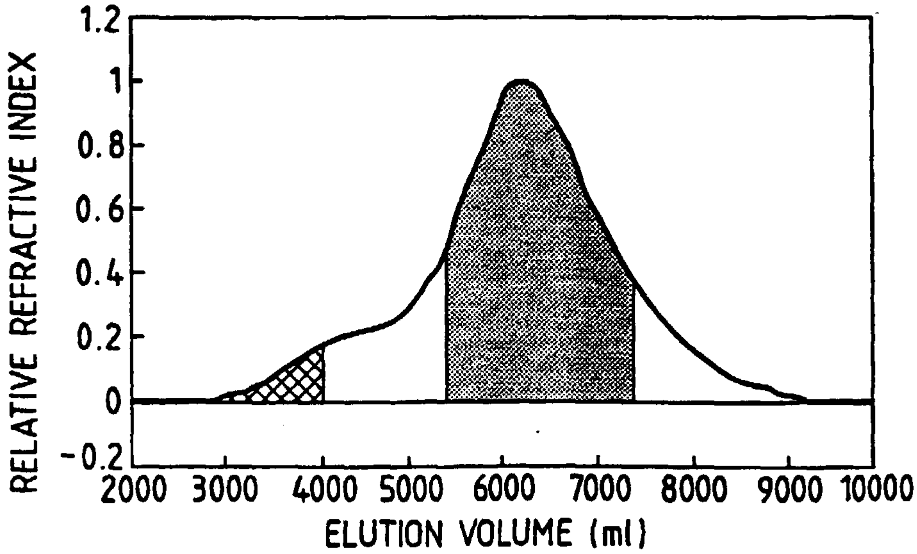

Fig.1d.

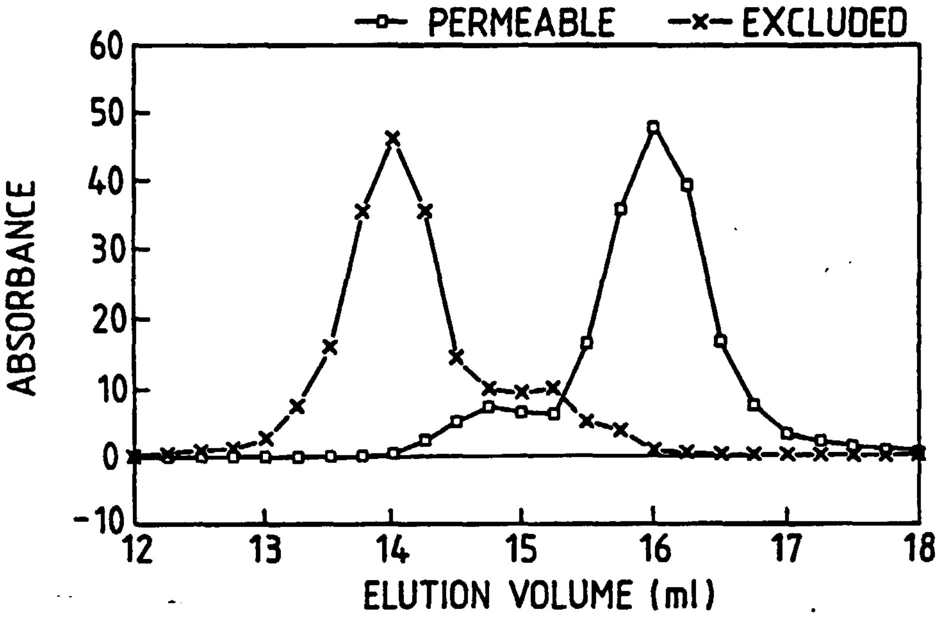
PERMEABLE
EXCLUDED
ABSORBANCE
60
50
40
30
20
10
0
-10
12
13
14
15
16
17
18
ELUTION VOLUME (ml)

Fig.1e.

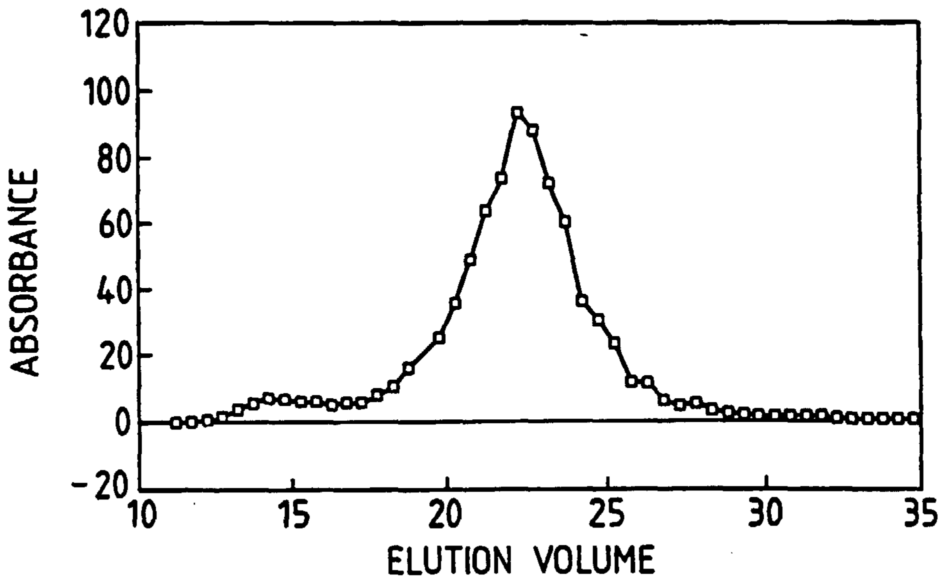
ABSORBANCE
120
100
80
60
40
20
0
-20
10
15
20
25
30
35
ELUTION VOLUME

Fig.2.

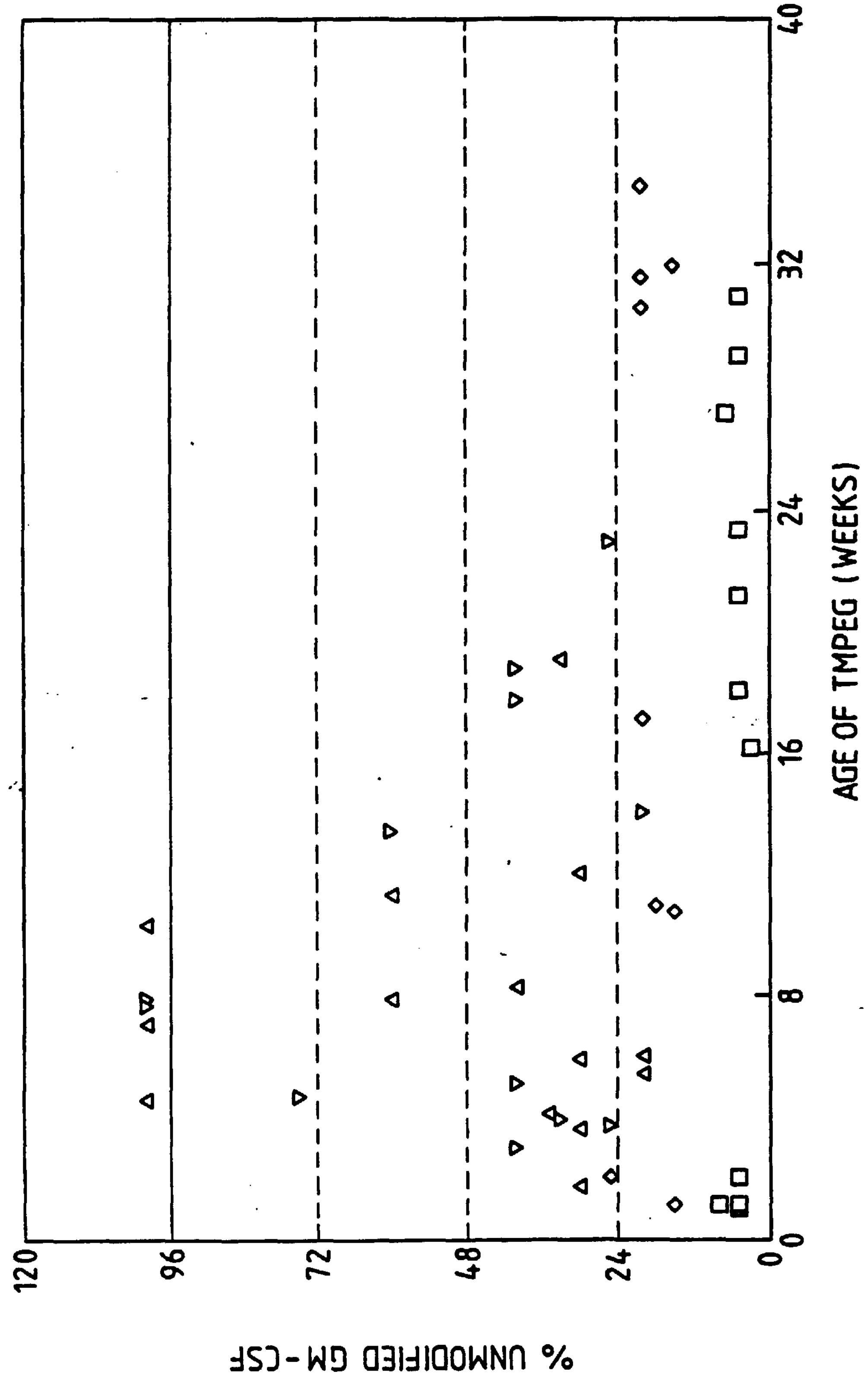
% UNMODIFIED GM-CSF
120
96
72
48
24
0
0
8
16
24
32
40
AGE OF TMPEG (WEEKS)

Fig.3a.

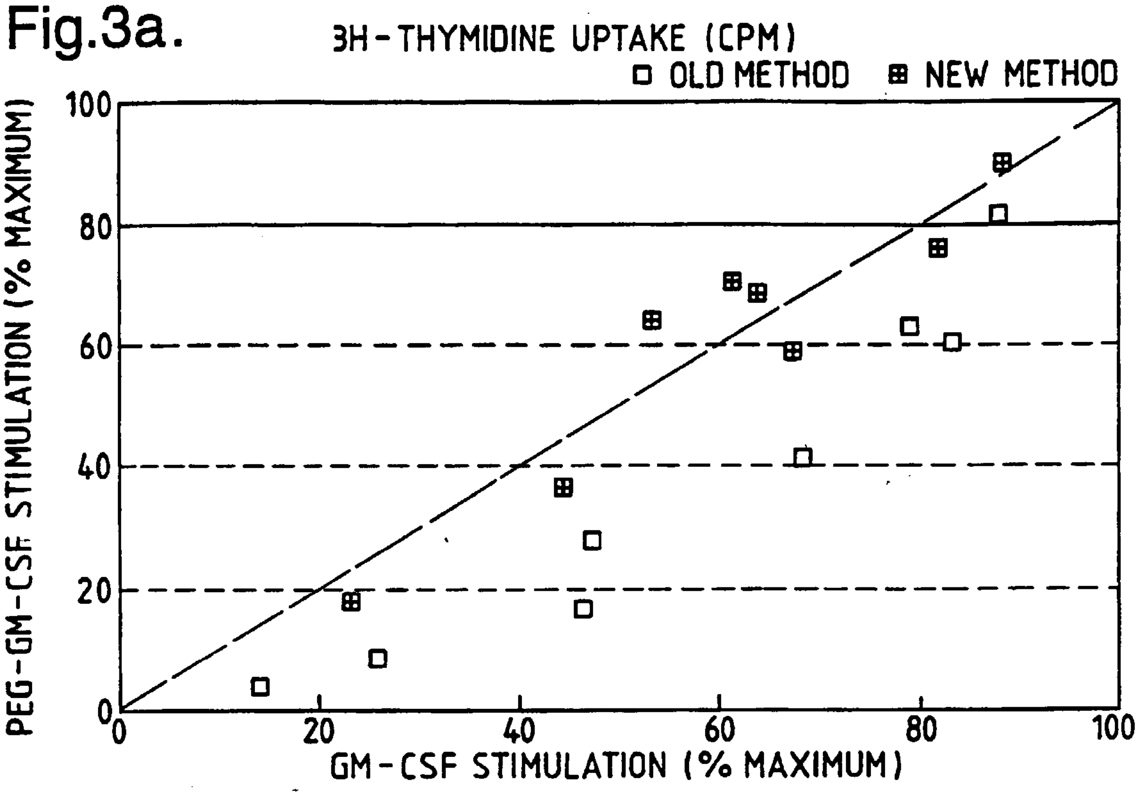
3H-THYMIDINE UPTAKE (CPM)
OLD METHOD
NEW METHOD
PEG-GM-CSF STIMULATION (% MAXIMUM)
100
80
60
40
20
0
0
20
40
60
80
100
GM-CSF STIMULATION (% MAXIMUM)

Fig.3b.

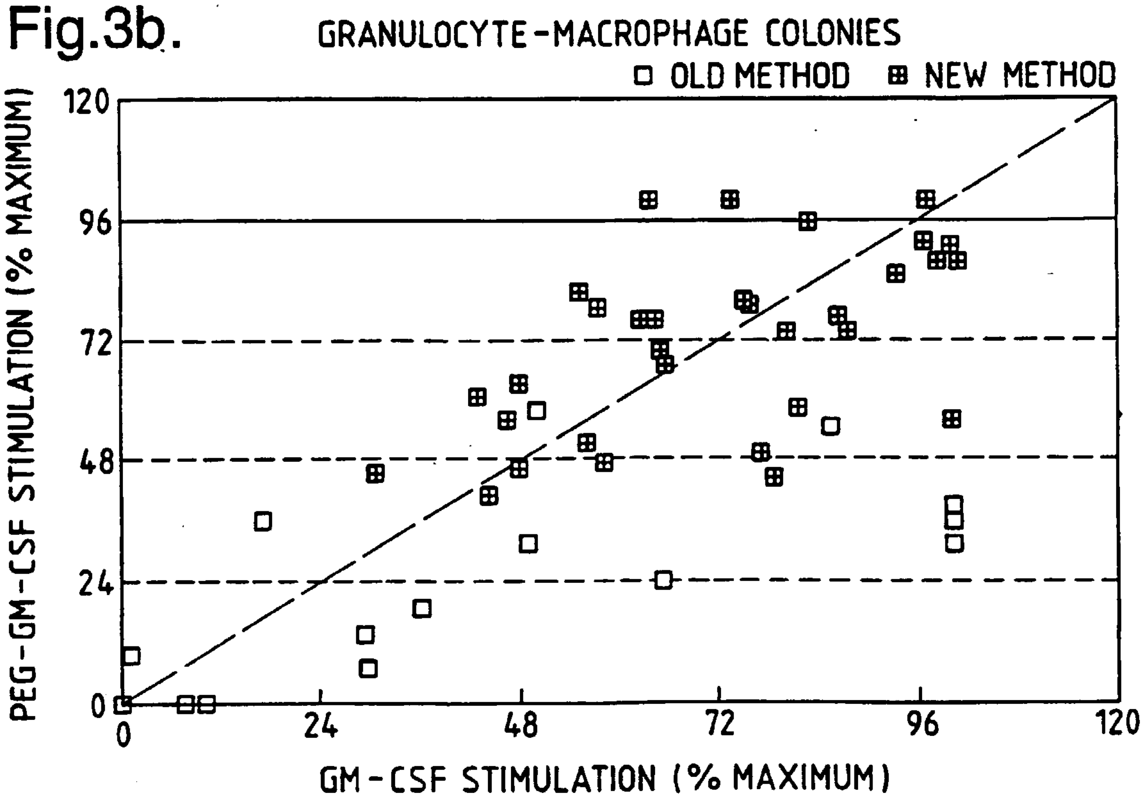
GRANULOCYTE-MACROPHAGE COLONIES
OLD METHOD
NEW METHOD
PEG-GM-CSF STIMULATION (% MAXIMUM)
120
96
72
48
24
0
0
24
48
72
96
120
GM-CSF STIMULATION (% MAXIMUM)

Fig.4a.

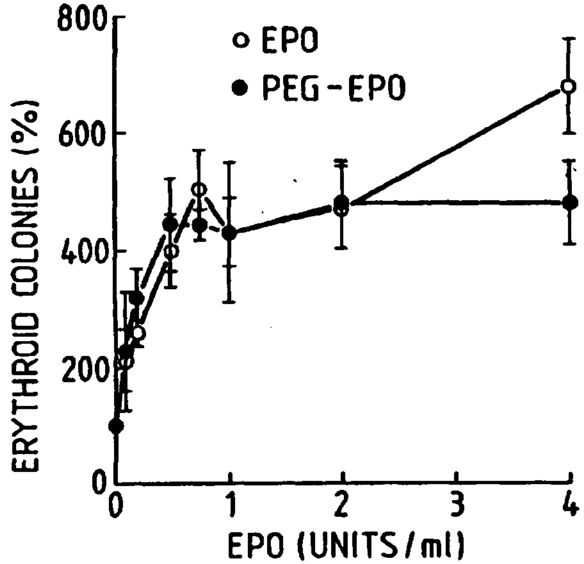


Fig.4b.

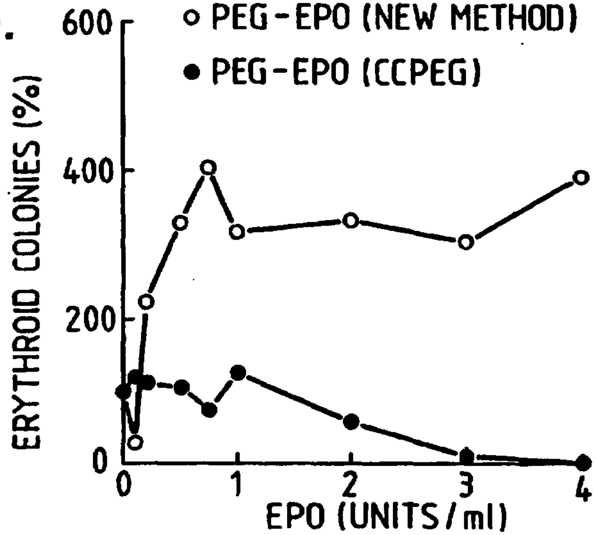


Fig.4c.

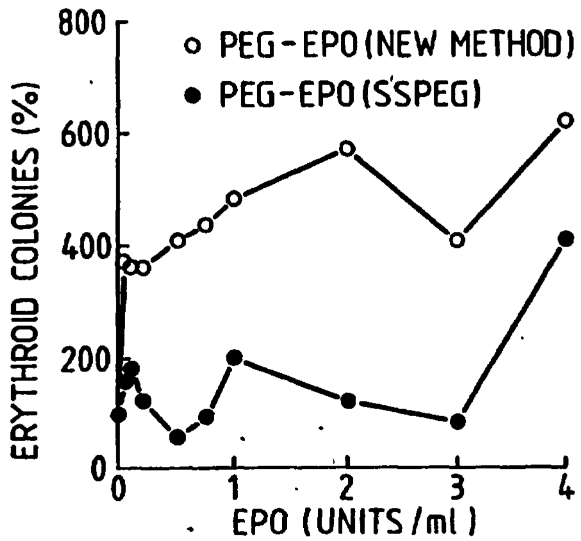


Fig.4d.

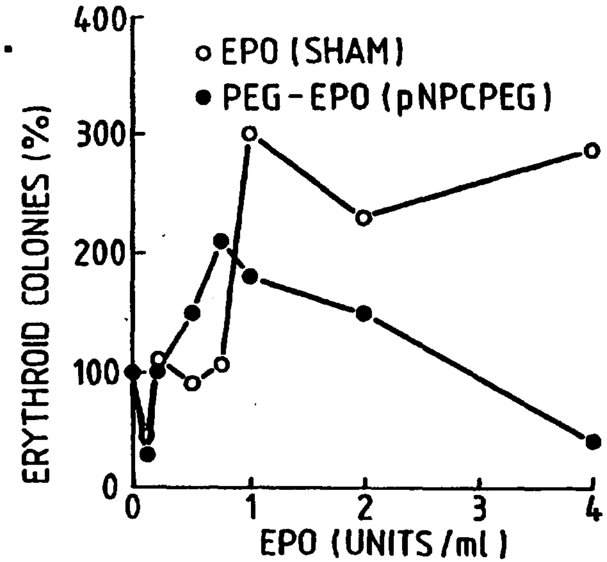

Fig.5.

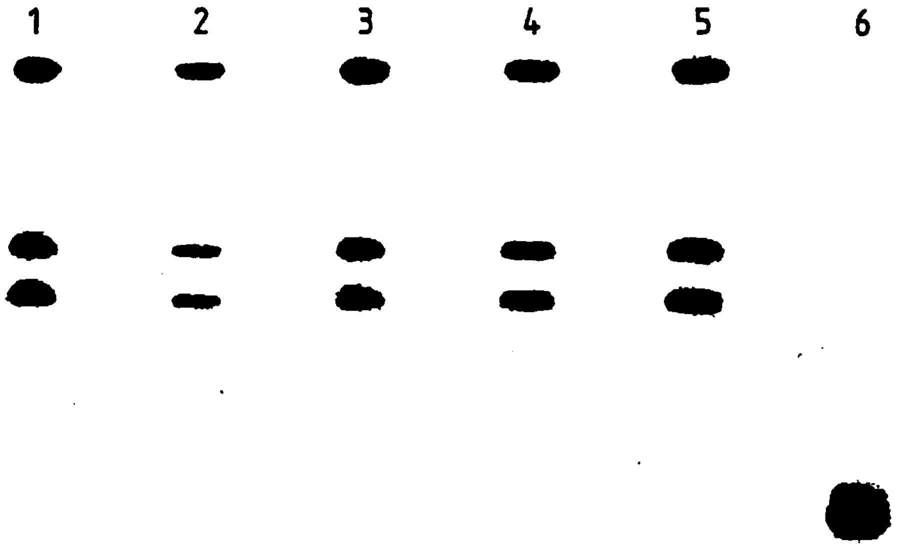
1
2
3
4
5
6

Fig.6a.

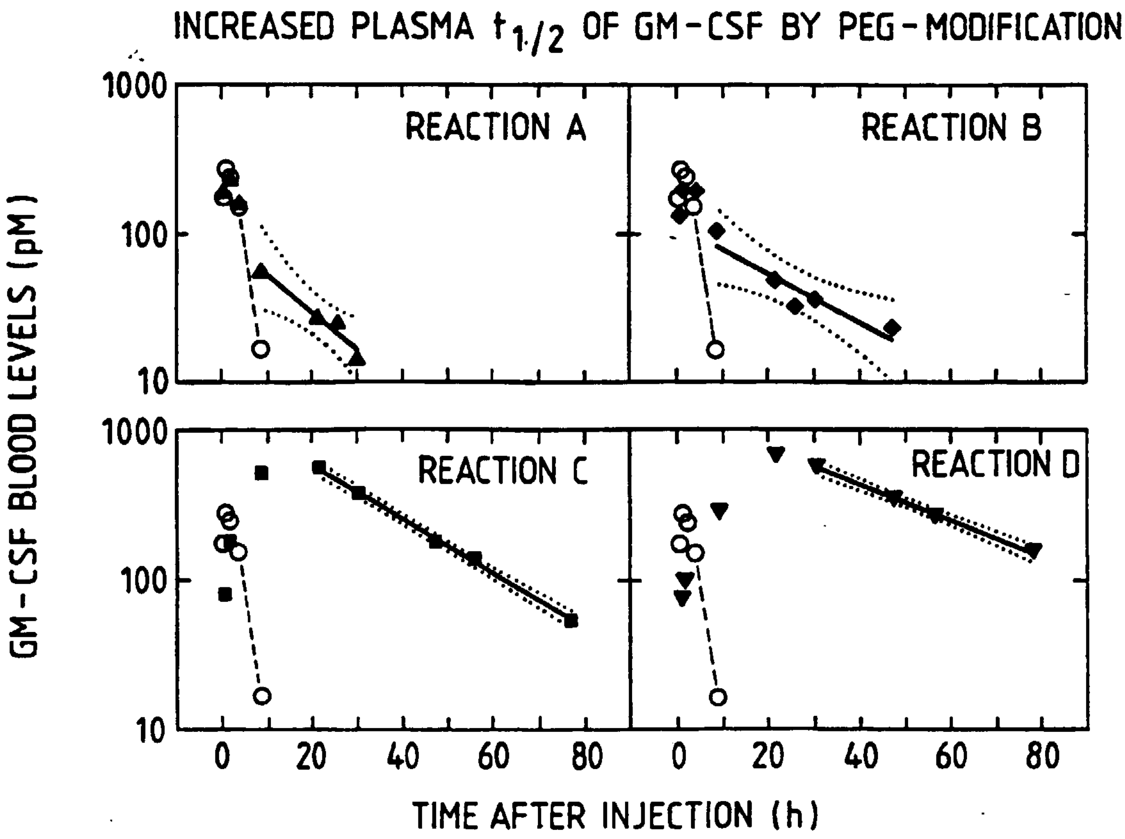
INCREASED PLASMA $t_{1/2}$ OF GM-CSF BY PEG-MODIFICATION
REACTION A
REACTION B
REACTION C
REACTION D
1000
100
10
GM-CSF BLOOD LEVELS (pM)
0
20
40
60
80
TIME AFTER INJECTION (h)

Fig.6b.

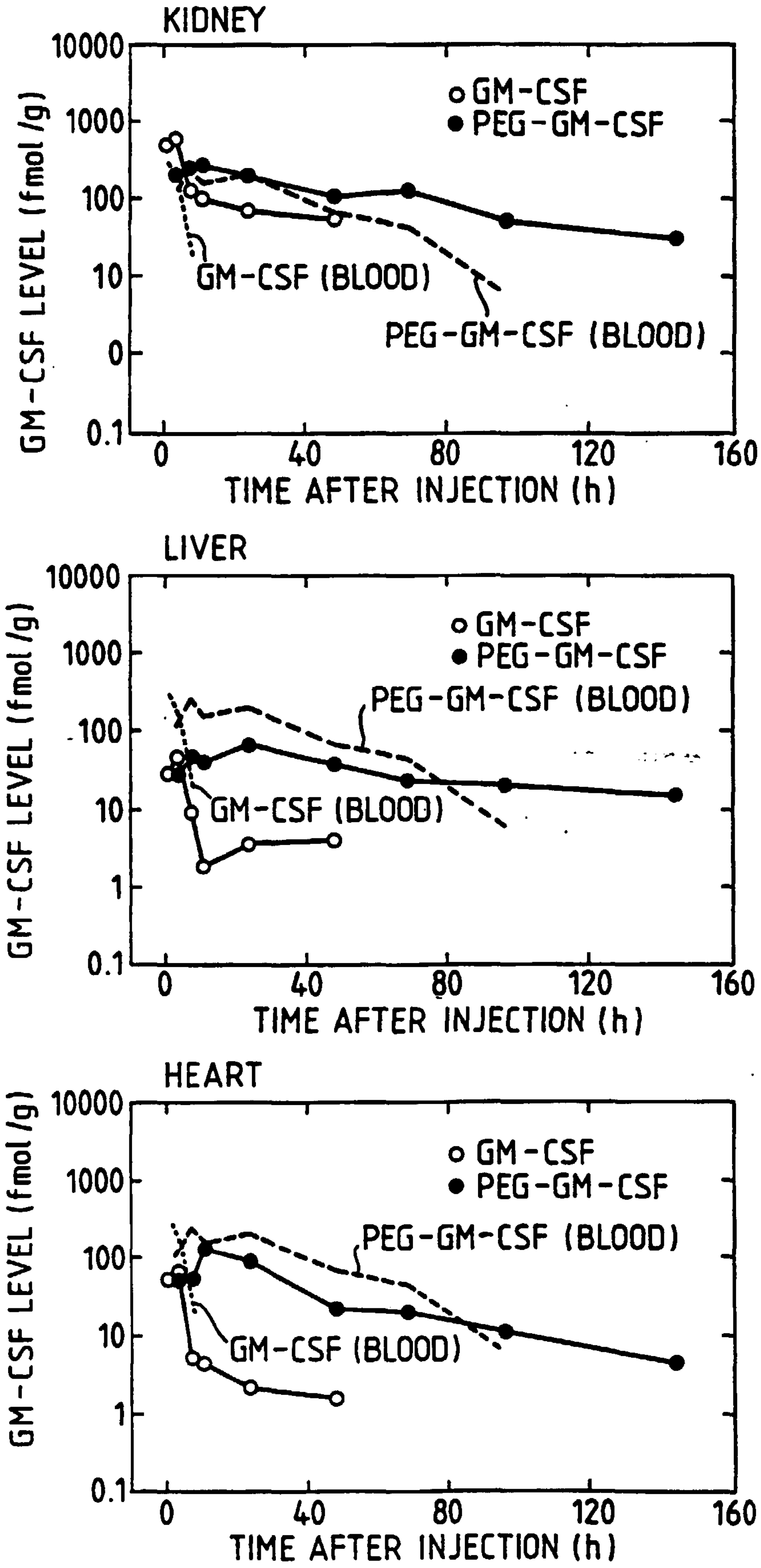
KIDNEY
GM-CSF LEVEL (fmol /g)
10000
1000
100
10
0
0.1
0
40
80
120
160
TIME AFTER INJECTION (h)
o GM-CSF
● PEG-GM-CSF
GM-CSF (BLOOD)
PEG-GM-CSF (BLOOD)
LIVER
GM-CSF LEVEL (fmol /g)
10000
1000
100
10
1
0.1
0
40
80
120
160
TIME AFTER INJECTION (h)
o GM-CSF
● PEG-GM-CSF
PEG-GM-CSF (BLOOD)
GM-CSF (BLOOD)
HEART
GM-CSF LEVEL (fmol /g)
10000
1000
100
10
1
0.1
0
40
80
120
160
TIME AFTER INJECTION (h)
o GM-CSF
● PEG-GM-CSF
PEG-GM-CSF (BLOOD)
GM-CSF (BLOOD)

# Fig.6b(Cont).

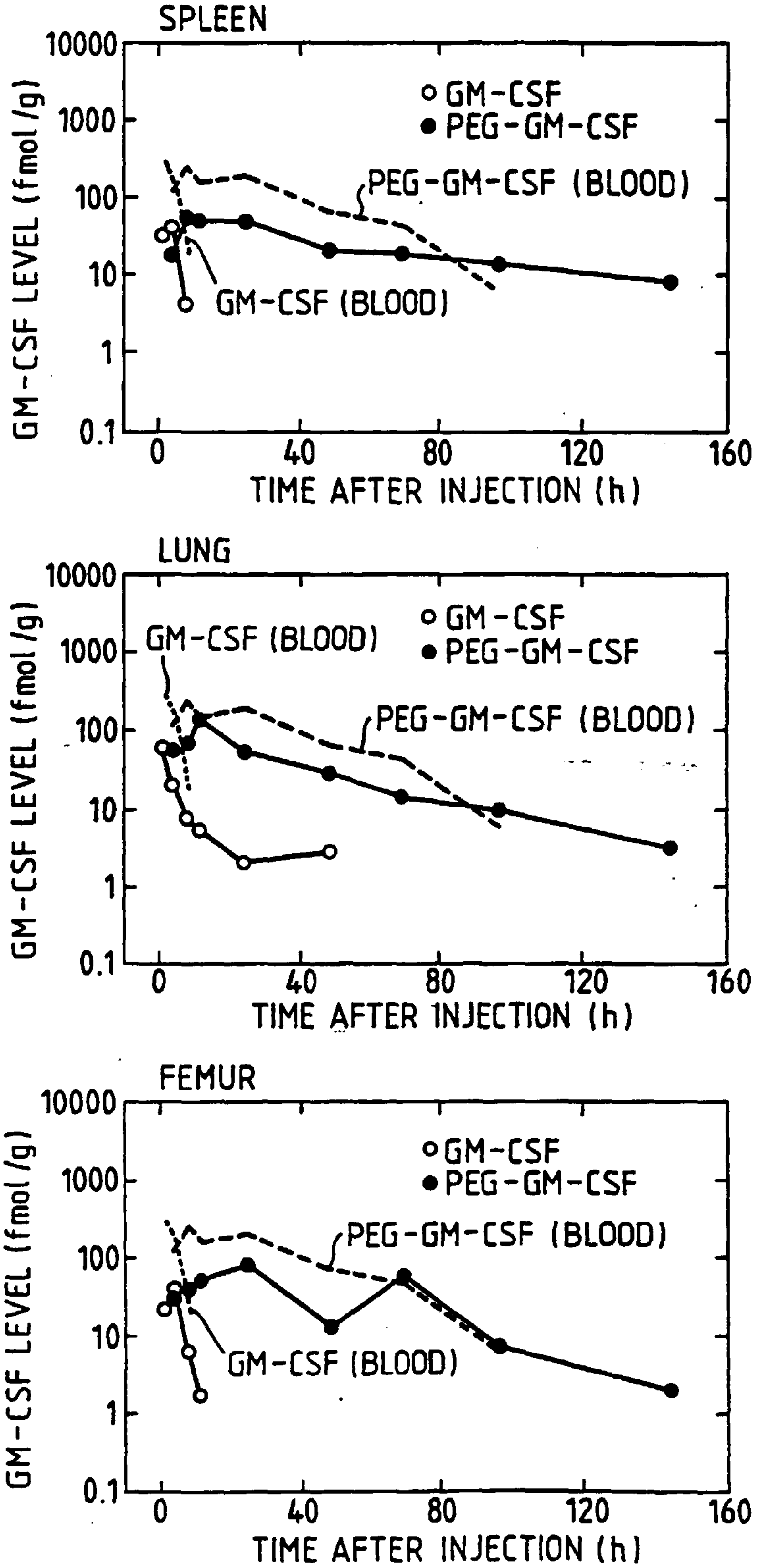
SPLEEN
GM-CSF LEVEL (fmol/g)
10000
1000
100
10
1
0.1
0
40
80
120
160
TIME AFTER INJECTION (h)
○ GM-CSF
● PEG-GM-CSF
PEG-GM-CSF (BLOOD)
GM-CSF (BLOOD)
LUNG
GM-CSF LEVEL (fmol/g)
10000
1000
100
10
1
0.1
0
40
80
120
160
TIME AFTER INJECTION (h)
GM-CSF (BLOOD)
○ GM-CSF
● PEG-GM-CSF
PEG-GM-CSF (BLOOD)
FEMUR
GM-CSF LEVEL (fmol/g)
10000
1000
100
10
1
0.1
0
40
80
120
160
TIME AFTER INJECTION (h)
○ GM-CSF
● PEG-GM-CSF
PEG-GM-CSF (BLOOD)
GM-CSF (BLOOD)

Fig.7.

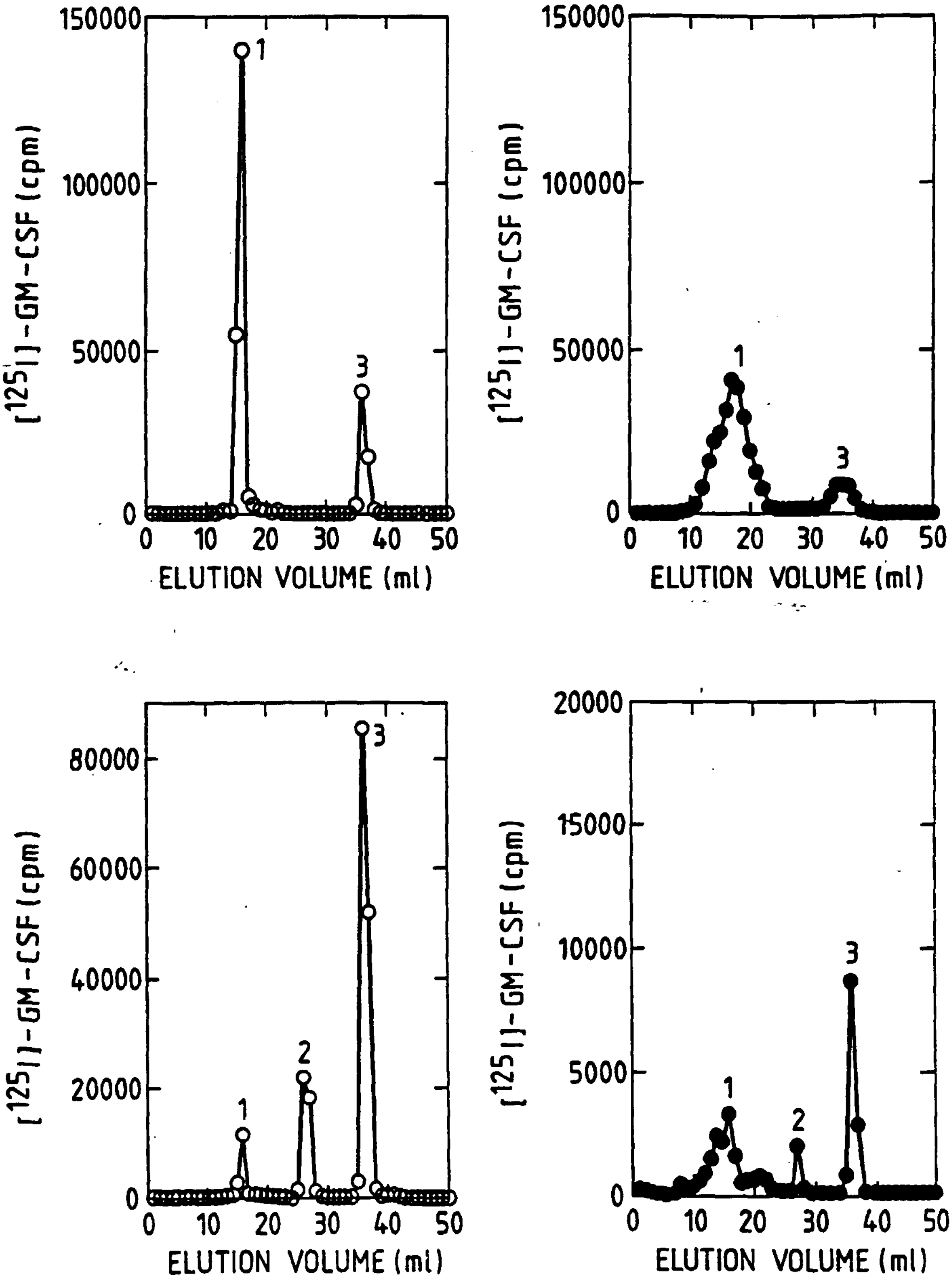

Fig.8a.

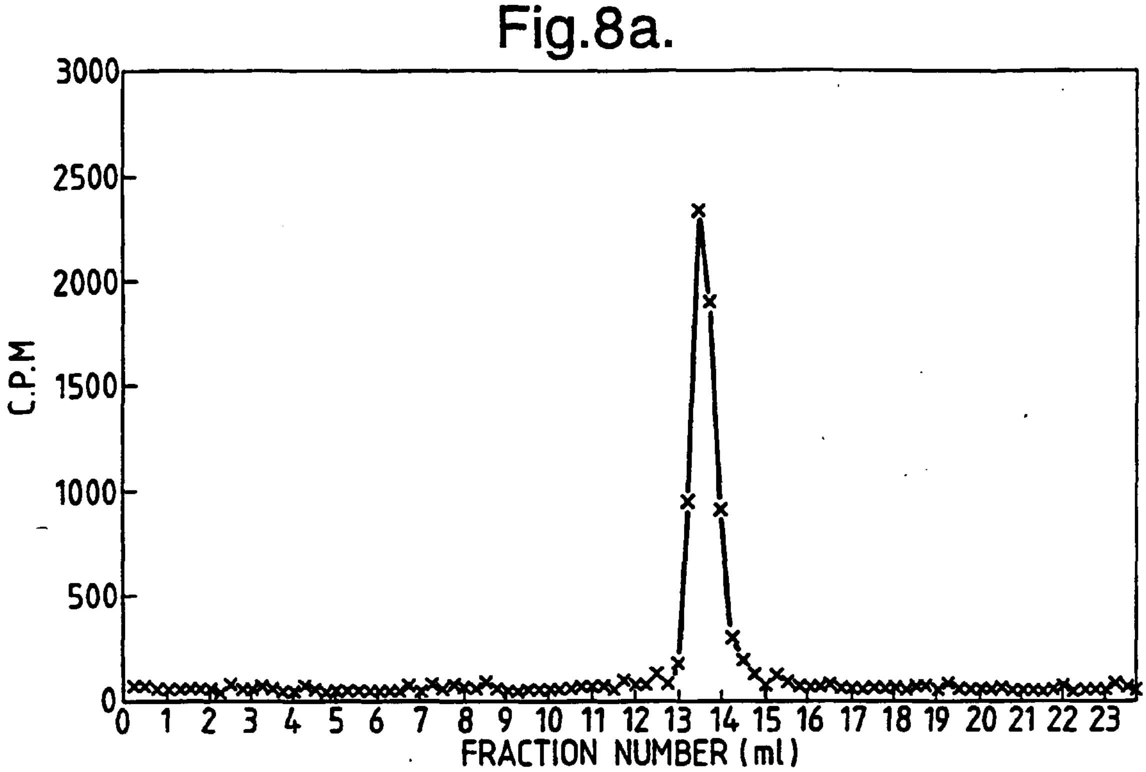
C.P.M
3000
2500
2000
1500
1000
500
0
0 1 2 3 4 5 6 7 8 9 10 11 12 13 14 15 16 17 18 19 20 21 22 23
FRACTION NUMBER (ml)

Fig.8b.

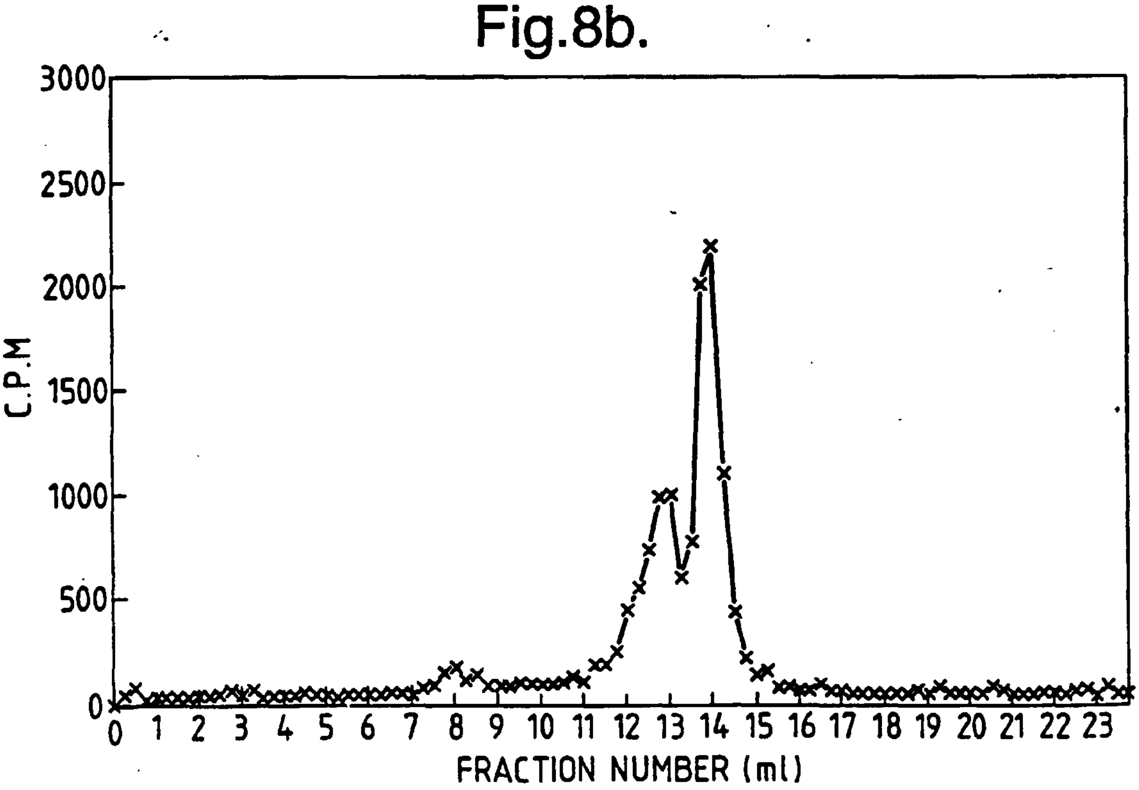
C.P.M
3000
2500
2000
1500
1000
500
0
0 1 2 3 4 5 6 7 8 9 10 11 12 13 14 15 16 17 18 19 20 21 22 23
FRACTION NUMBER (ml)

Fig.8c.

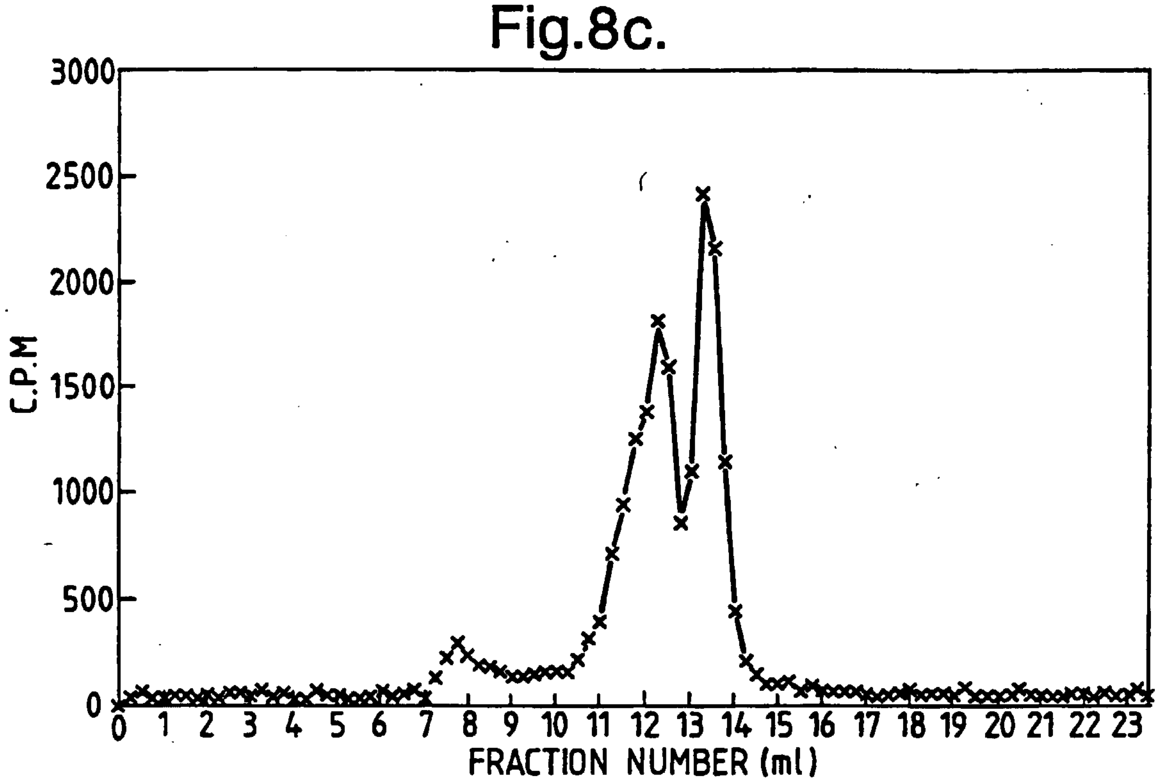
3000
2500
2000
1500
1000
500
0
C.P.M
0 1 2 3 4 5 6 7 8 9 10 11 12 13 14 15 16 17 18 19 20 21 22 23
FRACTION NUMBER (ml)

Fig.8d.

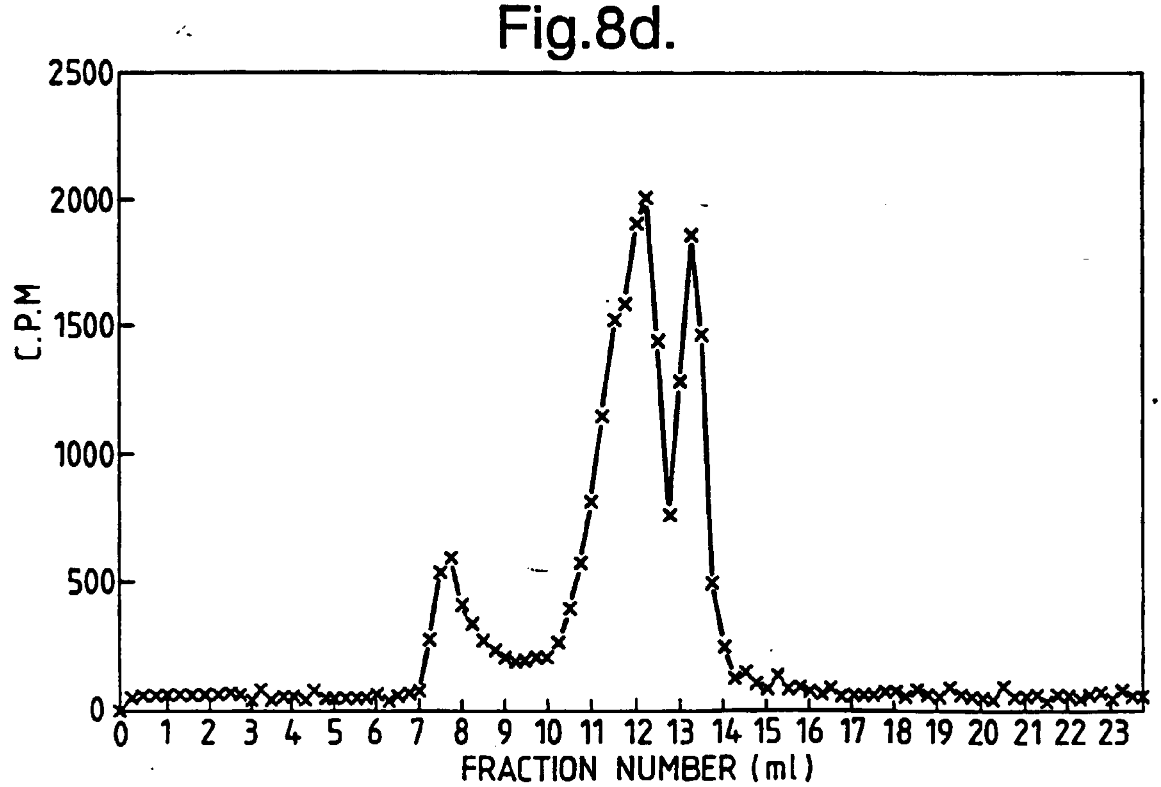
2500
2000
1500
1000
500
0
C.P.M
0 1 2 3 4 5 6 7 8 9 10 11 12 13 14 15 16 17 18 19 20 21 22 23
FRACTION NUMBER (ml)

Fig.8e.

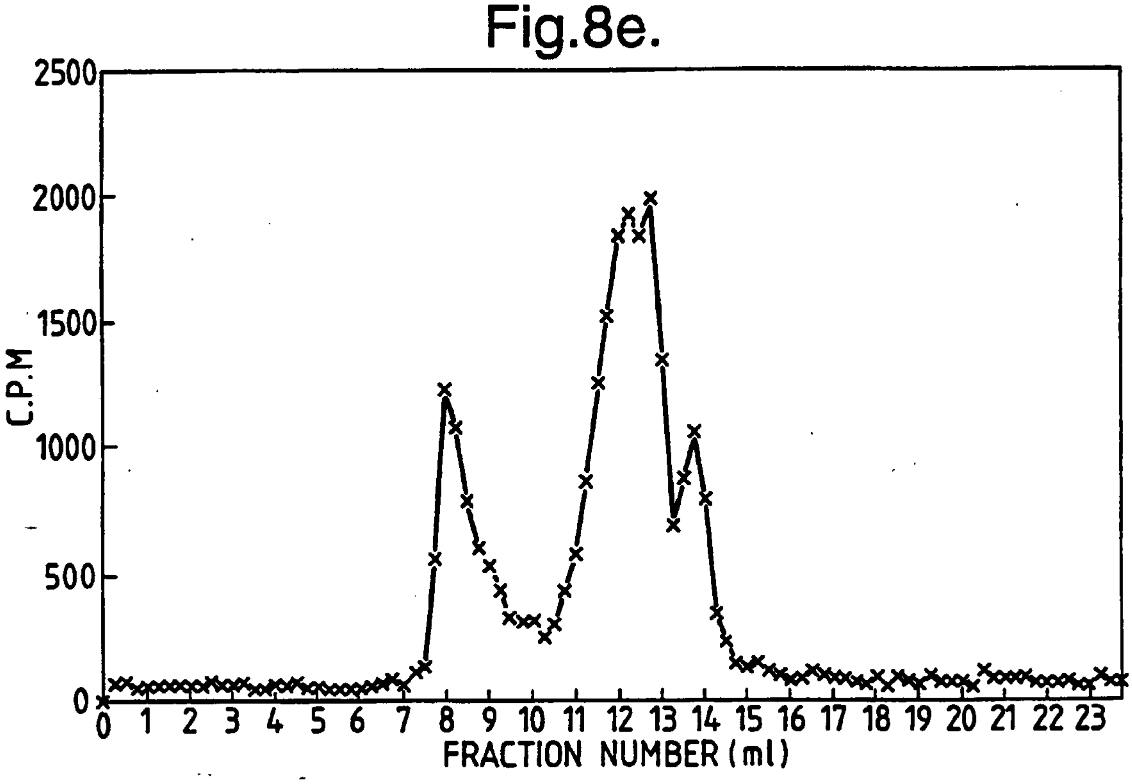
2500
2000
1500
1000
500
0
C.P.M
0 1 2 3 4 5 6 7 8 9 10 11 12 13 14 15 16 17 18 19 20 21 22 23
FRACTION NUMBER (ml)

Fig.8f.

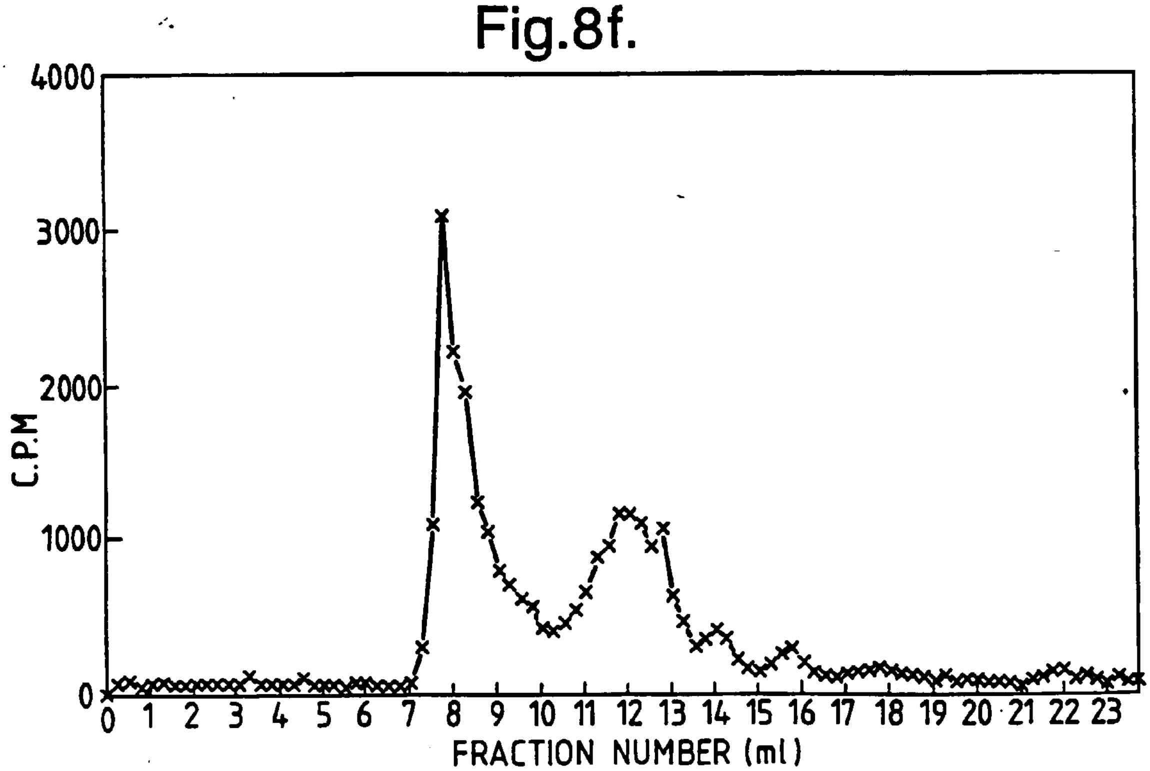
4000
3000
2000
1000
0
C.P.M
0 1 2 3 4 5 6 7 8 9 10 11 12 13 14 15 16 17 18 19 20 21 22 23
FRACTION NUMBER (ml)

Fig.9a.

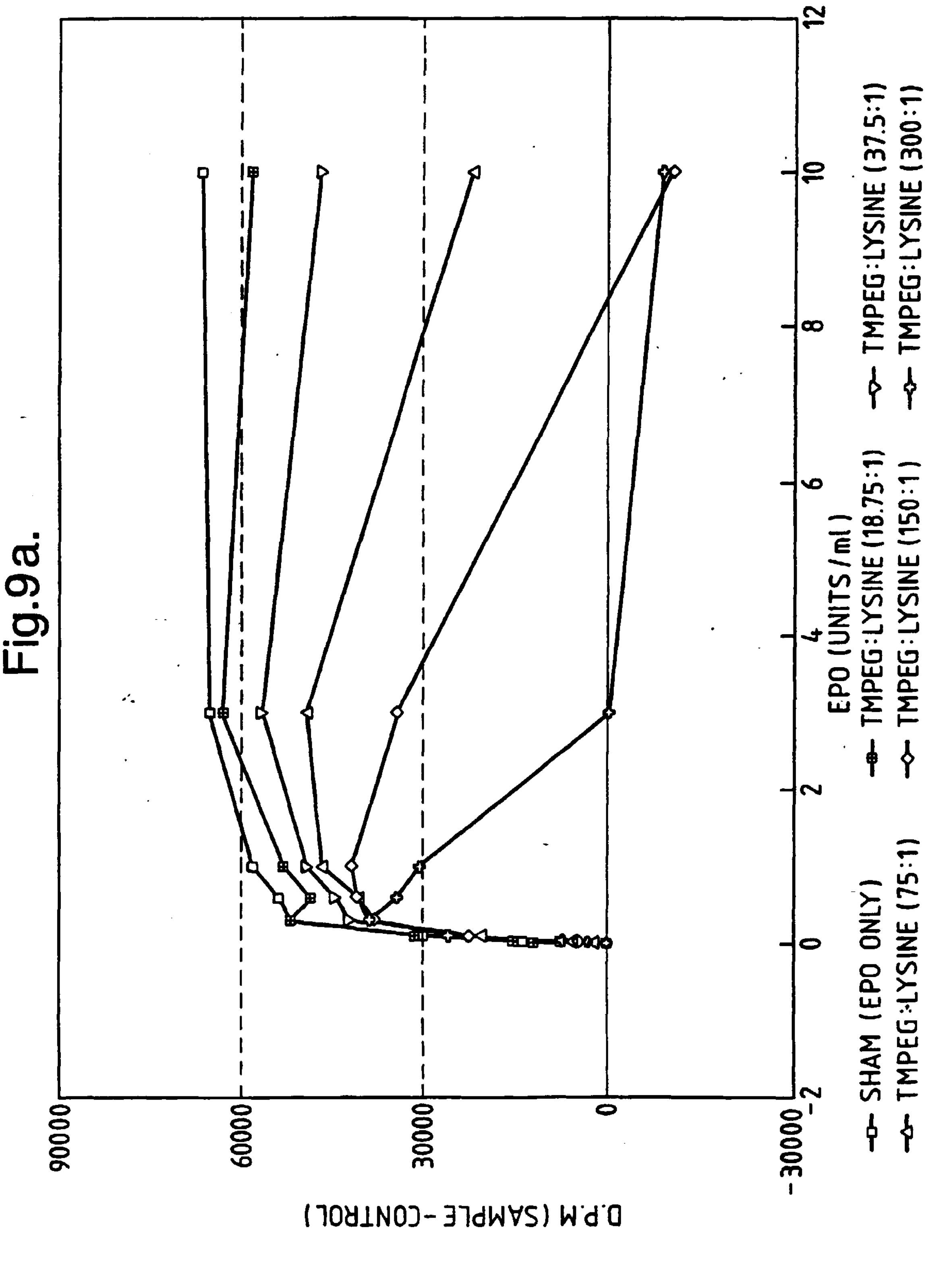
D.P.M (SAMPLE - CONTROL)
90000
60000
30000
0
-30000
-2
0
2
4
6
8
10
12
EPO (UNITS / ml)
SHAM (EPO ONLY)
TMPEG:LYSINE (18.75:1)
TMPEG:LYSINE (37.5:1)
TMPEG:LYSINE (75:1)
TMPEG:LYSINE (150:1)
TMPEG:LYSINE (300:1)

Fig.9b.

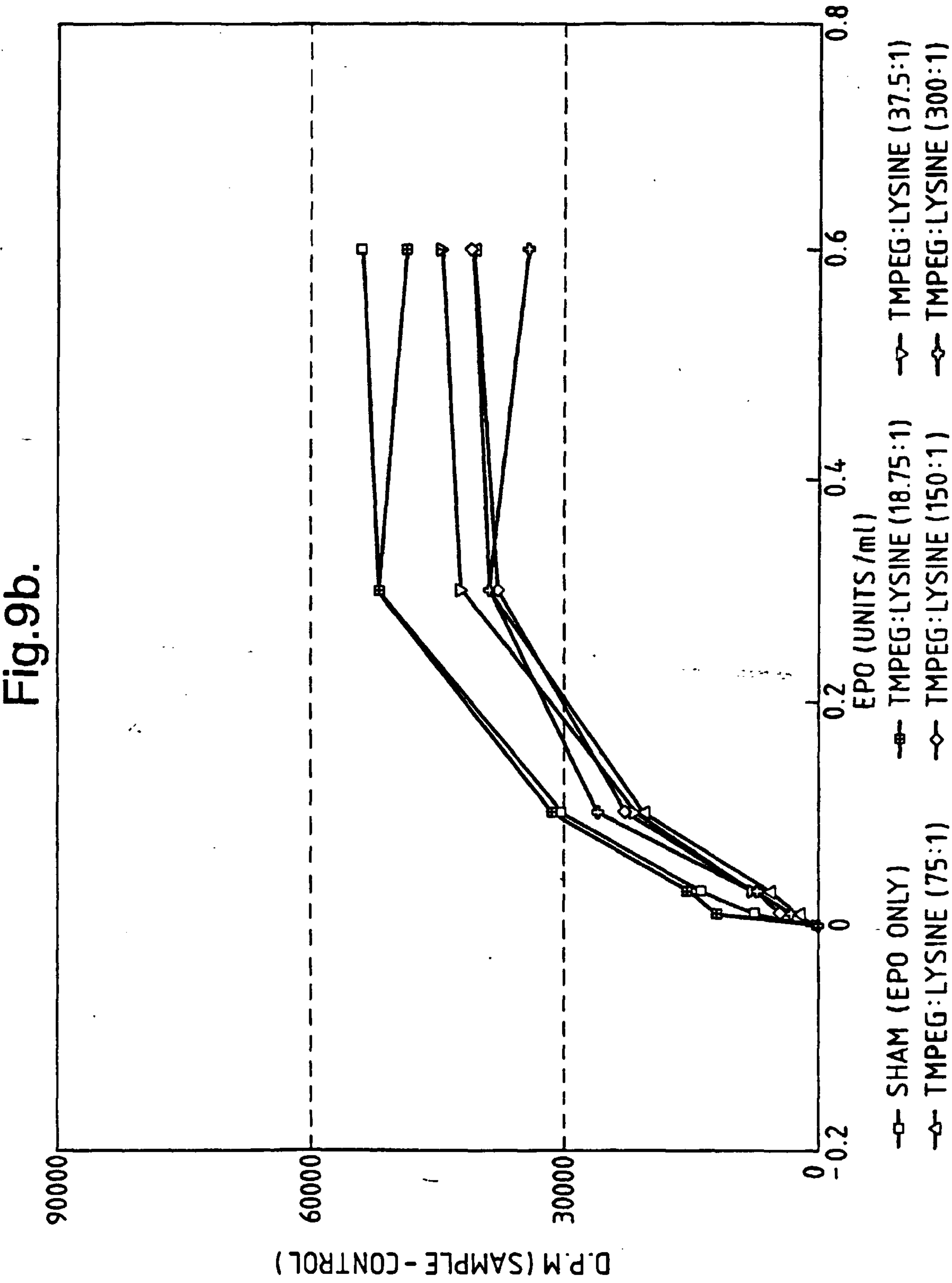
D.P.M (SAMPLE - CONTROL)
90000
60000
30000
0
-0.2
0
0.2
0.4
0.6
0.8
EPO (UNITS/ml)
SHAM (EPO ONLY)
TMPEG:LYSINE (18.75:1)
TMPEG:LYSINE (37.5:1)
TMPEG:LYSINE (75:1)
TMPEG:LYSINE (150:1)
TMPEG:LYSINE (300:1)

European Patent Office

# EUROPEAN SEARCH REPORT

Application Number
EP 00 10 5847

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
| --- | --- | --- | --- |
| D,X | WO 90 04606 A (ROYAL FREE HOSPITAL SCHOOL OF MEDICINE) 3 May 1990 (1990-05-03) * page 14 - page 16; table II * --- | 1-10 | C07K1/10<br>C07K1/13<br>A61K49/00<br>A61K47/48<br>A61K9/127 |
| D,X | WO 90 04650 A (ROYAL FREE HOSPITAL SCHOOL OF MEDICINE) 3 May 1990 (1990-05-03) * page 7 - page 9 * --- | 110 | |
| D,X | WO 90 04384 A (ROYAL FREE HOSPITAL SCHOOL OF MEDICINE) 3 May 1990 (1990-05-03) * page 3, line 8,9 * --- | 1-10 | |
| X | F.MALIK ET AL.: "Polyethylene Glycol (PEG)- modified Granulocyte-Macrophage Colony- stimulating Factor (GM-CSF) with conserved Biological Activity" EXP.HEMATOL., vol. 20, 1992, pages 1028-35, XP002138685 *Materials and Methods* --- | 1-10 | |
| X | C.DELAGO ET AL.: "Coupling of Poly(ethylene glycol) to Albumin under Very Mild Conditions by Activation with Tresyl Chloride: Characterization of the Conjugate by Partitioning in Aqueous Two-Phase Systems" BIOTECHNOL.APPL.BIOCHEM., vol. 12, no. 2, 1990, pages 119-128, XP002138686 *Materials and Methods* --- -/-- | 1-10 | TECHNICAL FIELDS SEARCHED (Int.Cl.7)<br>C07K<br>A61K |

The present search report has been drawn up for all claims

| Place of search | Date of completion of the search | Examiner |
| --- | --- | --- |
| MUNICH | 25 May 2000 | Deffner, C-A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 00 10 5847

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | J.SENIOR ET AL.: "Influence of surface hydrophilicty of liposomes on their interaction with plasma protein and clearance from the circulation: studies with poly(ethylene glycol)-coated vesicles" BIOCHEM.BIOPHYS.ACTA, vol. 106, 1991, pages 77-82, XP002138687 NETHERLAND * the whole document * --- | 1-10 | |
| X | C.KNÜSLI ET AL.: "Polyethylene glycol (PEG) modification of granulocyte-macrophage colony stimulating factor (GM-CSF) enhances neutrophil priming activity but not colony stimulating activity" BRITHISH JOURNAL OF HAEMATOLOGY, vol. 82, 1992, pages 654-663, XP002138688 * figures 1-4 * --- | 1-10 | |
| X | K.NILSSON ET AL.: "High-Performance Liquid Chromatography on Silica-Bound Alcohol Dehydrogenase" ANALYTICAL.BIOCHEM., vol. 134, 1983, pages 60-72, XP002139689 *Materials and Methods* --- | 1-10 | TECHNICAL FIELDS SEARCHED (Int.Cl.7) |
| X | C.DELAGO ET AL.: "Immunoaffinity cell partitioning of erythrocytes" BIOCHEM.SOC.TRANS., vol. 16, no. 6, 1988, pages 968-969, XP002138690 * the whole document * --- | 1-10 | |
| X | EP 0 539 167 A (ORTHO PHARMA CORP) 28 April 1993 (1993-04-28) * tables 2,3 * ----- | 10 | |

The present search report has been drawn up for all claims

| Place of search | Date of completion of the search | Examiner |
|---|---|---|
| MUNICH | 25 May 2000 | Deffner, C-A |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)